# EUROPÄISCHE PATENTSCHRIFT

(11) **EP 1 926 743 B1**
(45) Veröffentlichungstag und Bekanntmachung des Hinweises auf die Patenterteilung: **15.05.2013**
(21) Anmeldenummer: 06805748.8
(22) Anmeldetag: 18.09.2006
(51) Int. Cl.: C07K 5/06, A61K 38/55, A61P 7/02, A61P 9/00

(54) **2-(aminomethyl)-5-chlor-benzylamid-Derivate und ihre Verwendung als Hemmstoffe des Gerinnungsfaktors Xa**
2-(aminomethyl)-5-chlorobenzylamide derivatives and their use as inhibitors of the clotting factor Xa
Derivés du 2-(aminomethyl)-5-chloro-benzylamide et leur utilisation comme inhibiteurs du facteur de coagulation Xa

(30) Priorität: 16.09.2005 DE 102005044319
(43) Veröffentlichungstag der Anmeldung: 04.06.2008
(73) Patentinhaber: The Medicines Company (Leipzig) GmbH, 04103 Leipzig (DE)
(72) Erfinder: STEINMETZER, Torsten, 07743 Jena (DE); DÖNNECKE, Daniel, Victoria BC, V8Z 5L8 (CA); SCHWEINITZ, Andrea, 07749 Jena (DE); STÜRZEBECHER, Anne, 99425 Weimar (DE); STÜRZEBECHER, Jörg, 99094 Erfurt (DE)
(74) Vertreter: Bösl, Raphael Konrad
(86) Internationale Anmeldenummer: PCT/EP2006/009052
(87) Internationale Veröffentlichungsnummer: WO 2007/031343

(56) Entgegenhaltungen:
- WO-A-02/50056
- WO-A-03/076457
- WO-A2-02/06280
- MATTHEW M MORRISSETTE ET AL.: "Low molecular weight thrombin inhibitors with excellent potency, metabolic stability, and oral bioavailability" BIOORGANIC AND MEDICINAL CHEMISTRY LETTERS, Bd. 14, 16. August 2004 (2004-08-16), Seiten 4161-4164, XP002408310
- KENNETH E RITTLE ET AL.: "Unexpected Enhancement of Thrombin Inhibitor Potency with o-Aminoalkylbenzylamides in the P1 Position" BIOORGANIC AND MEDICINAL CHEMISTRY LETTERS, Bd. 13, 20. Oktober 2003 (2003-10-20), Seiten 3477-3482, XP002408311
- HERBERT NAR ET AL.: "Structural Basis for Inhibition Promiscuity of Dual Specific Thrombin and Factor Xa Blood Coagulation Inhibitors" STRUCTURE, Bd. 9, Nr. 1, Januar 2001 (2001-01), Seiten 29-37, XP002408312

## Beschreibung

Die Erfindung betrifft neue Hemmstoffe des Gerinnungsfaktors Xa (FXa), ihre Herstellung und auch solche Hemmstoffe zur Verwendung für Arzneimittel und zur Therapie, Prophylaxe und Diagnose von kardiovaskulären Erkrankungen und thromboembolischen Ereignissen.

Die gegenwärtig klinisch eingesetzten Antikoagulantien vom Heparin-Typ bzw. die Vitamin-K-Antagonisten werden nicht allen Anforderungen an ein "ideales" Anti-thrombotikum gerecht. Deshalb wird mit niedermolekularen Hemmstoffen der Gerinnungsenzyme, speziell von Thrombin und FXa, nach Alternativen gesucht. Ein besonderer Vorteil von FXa-Hemmstoffen im Vergleich zu Thrombin-Hemmstoffen könnte die geringere Blutungsneigung sein, die sich bei verschiedenen Tierversuchen gezeigt hat. So wird bei antithrombotisch effektiven Dosen im Falle der Anwendung von FXa-Hemmstoffen die Blutungszeit nur minimal beeinflußt (Leadley, R.J. Curr. Topics in Med. Chemistry 1, 151 (2001); sowie Quan, M.L. & Smallheer, J.M., Curr. Opin. in Drug Discovery & Development 7, 460 (2004)).

Inzwischen wurden verschiedene wirksame FXa-Hemmstoffe entwickelt (Quan, M.L. & Smallheer, J.M., Curr. Opin. In Drug Discovery & Development 7, 460 (2004); Pauls, H.W. et al., Frontiers in Medicinal Chemistry - Online 1, 129 (2004), sowie Maignan, S. & Mikol, V. Curr. Topics in Med. Chemistry 1, 161 (2001)). Die ersten beschriebenen FXa-Hemmstoffe enthielten in ihrem C-terminalen Segment stark basische Gruppierungen, wie beispielsweise Amidino- oder Guanidinogruppen.

Durch Röntgenstrukturanalysen wurde gezeigt, dass diese basischen Gruppen eine Salzbrücke mit der für trypsinartige Serinproteasen charkteristischen Asparaginsäure in Position 189 des FXa ausbilden. Aufgrund dieser stark geladenen basischen Gruppen zeigten die FXa-Hemmstoffe der ersten Generation in der Regel nur eine sehr geringe Bioverfügbarkeit nach oraler Applikation. Deshalb wurde in den letzten Jahren intensiv nach neuen FXa-Hemmstoffen gesucht, die in Ihrem C-terminalen Segment keinerlei basische Gruppe mehr besitzen. Eine weitere Strategie bestand in der Entwicklung von Inhibitoren mit verminderter Basizität, die unter physiologischen pH-Bedingungen nur teilweise protoniert sind. Eine dritte Strategie war die Entwicklung von Prodrugs, bei denen die basischen Gruppen erst nach oraler Aufnahme im Körper freigesetzt werden (Quan, M.L. & Smallheer, J.M., Curr. Opin. In Drug Discovery & Development 7, 460 (2004); Pauls, H.W. et al., Frontiers in Medicinal Chemistry - Online 1, 129 (2004), sowie Maignan, S. & Mikol, V. Curr. Topics in Med. Chemistry 1, 161 (2001)).

Obwohl inzwischen erste oral verfügbare FXa-Inhibitoren in die klinische Entwicklung überführt wurden (Perzbom, E., J. of Thromb. & Haemost. 3, 514 (2005); Quan, M.L., J. Med. Chem. 48, 1729 (2005)), hat bisher noch kein direkter FXa-Hemmstoff die Zulassung erhalten. Deshalb wird derzeit weiter intensiv an der Entwicklung neuer FXa-Hemmstoffe gearbeitet.

Der Erfindung liegt daher die Aufgabe zu Grunde, einen für therapeutische Anwendungen geeigneten Wirkstoff anzugeben, der den Gerinnungsfaktor Xa mit hoher Aktivität und Spezifität hemmt und der vorzugsweise nach i.v.-, s.c.- oder oraler Gabe möglichst lange im Körper zirkuliert. Auch die Bereitstellung einer pharmazeutischen Zubereitung ist Gegenstand dieser Erfindung.

In den vergangenen Jahren ist es gelungen, substratanaloge Thrombinhemmstoffe mit einem C-terminalen 2-(Aminomethyl)-5-Chlor-Benzylamid zu entwickeln (Selnick, H.G. et al., WO 02/50056; Rittle, K.E. et al., Bioorg. Med. Chem. Lett. 13, 3477 (2003), Stauffer, K.J. et al., J. Med. Chem. 48, 2282 (2005)). Für mehrere der beschriebenen Verbindungen konnte eine signifikante Bioverfügbarkeit und antithrombotische Wirksamkeit nach oraler Gabe an verschiedenen Versuchstieren beobachtet werden. Mittels Röntgenstrukturanalyse wurde gezeigt, dass die freie Aminomethylgruppe des C-terminalen 2-(Aminomethyl)-5-Chlor-Benzylamid-Restes eine Salzbrücke mit dem für Thrombin charakteristischen Glutaminsäurerest 192 ausbildet und dadurch signifikant zur inhibitorischen Wirksamkeit beiträgt (Rittle, K.E. et al., Bioorg. Med. Chem. Lett. 13, 3477 (2003), Stauffer, K.J. et al., J. Med. Chem. 48, 2282 (2005)).

Aus WO 02/50056 sind Prolinamid-Derivate bekannt, die eine Thrombin-inhibierende Wirkung aufweisen und zur Therapie von Embolien und Thrombosen eingesetzt werden können. In der Veröffentlichung Bioorganic Medical Chemistry Letters (13, 2003, Seiten 3477-3482, K.E. Rittle et al.) werden Benzolsulfonamidopyridinon-Derivate beschrieben, die Thrombinhemmende Eigenschaften aufweisen.

Von uns wurde überraschenderweise gefunden, dass geeignet acyliertes 2-(Aminomethyl)-5-Chlor-Benzylamin gemäß der allgemeinen Formel I,

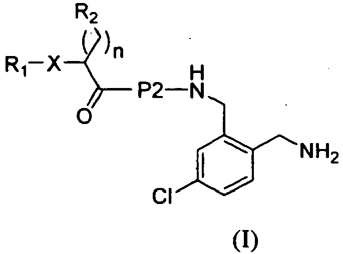

FXa sehr wirksam und selektiv hemmt, obwohl FXa im Gegensatz zu Thrombin keine Glutaminsäure in Position 192 besitzt und daher die freie Aminomethylgruppe des C-terminalen Restes keine Salzbrücke mit dem Rest 192 ausbilden kann. Überraschenderweise zeigen besonders geeignete Verbindungen, die als P3-Rest (Nomenklatur nach Schechter und Berger, Biochem. Biophys. Res. Commun. 27, 157 1967)) ein Derivat des Homophenylalanins in D-Konfiguration besitzen, insbesondere Verbindungen mit einem P3-Rest der Struktur II 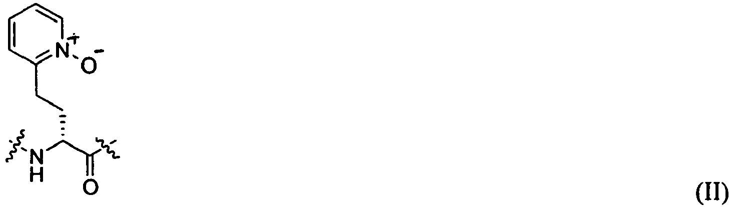 im Vergleich ihrer Wirkung auf FXa und Thrombin eine deutlich stärkere Hemmaktivität auf FXa. Als weitere geeignete P3-Reste erwiesen sich D-Homotyrosin oder D-Homopyridylalanin, wobei der Ringstickstoff in para-, meta-, oder ortho-Stellung stehen kann oder als N-Oxid vorliegt.

Ein Gegenstand der vorliegenden Erfindung ist daher eine Verbindung der allgemeinen Formel I 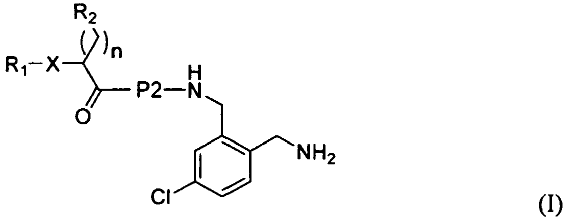 sowie pharmazeutisch geeignete Salze dieser Verbindungen als Hemmstoffe des Gerinnungsfaktors Xa, bei der
- X: = NR₃ oder O,
- R₃: = bevorzugt H, aber auch ein verzweigter bzw. unverzweigter Alkylrest mit 1-6 C-Atomen, insbesondere Alkyl mit 1-3 C-Atomen, vor allem Methyl sein kann; mit
- n: = 0, 1, 2, 3 oder 4, vorzugsweise mit n = 1 oder 2, insbesondere n=2;
- R₁: = H, -CH₂-COOR₄, -SO₂-R₅, -COOR₅ oder CO-R₆, insbesondere -SO₂-R₅;
- R₄: = H oder ein verzweigter bzw. unverzweigter Alkylrest mit 1-6 C-Atomen, insbesondere Alkyl mit 1 bis 3 C-Atomen, insbesondere Ethyl;
- R₅: = ein mit einem Rest R₇ substituierter oder unsubstituierter verzweigter oder unverzweigter Alkylrest mit 1-7 C-Atomen, insbesondere 1-4 C-Atomen, oder R₅ ein mit einem Rest R₇ substituierter oder unsubstituierter Aryl- oder Heteroarylrest, oder ein mit einem Rest R₇ substituierter oder unsubstituierter Aralkyl- oder Heteroaralkylrest, oder ein Cyclohexylmethylrest
- R₇: = Halogen, bevorzugt Cl oder F oder CN, NHR₃, NHCO-R₃, -CH₂-NHR₃, NO₂, OR₃, SR₃, -COOR₄ oder -CH₂-COOR₄ und R₃ sowie R₄ wie oben definiert
- R₆: = ein mit einem Rest R₇ substituierter oder unsubstituierter verzweigter bzw. unverzweigter Alkylrest mit 1-8 C-Atomen, insbesondere 1-4 C-Atomen, oder Cycloalkyl bzw. ein Cyclohexylmethyl, aber auch -COOR₄, wobei R₄ wie oben definiert ist; und auch R₆ mit R₇ substituiert sein kann, das wie oben definiert ist, und
- R₂ =: eine beliebige Struktur ausgewählt aus der Gruppe bestehend aus den folgenden Strukturen 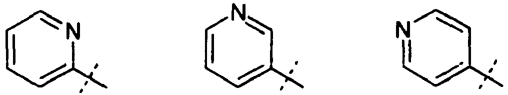 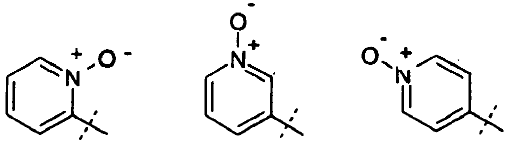
- P2 =: ein beliebiger natürlicher oder unnatürlicher α-Aminosäurerest folgender Struktur 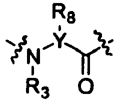 mit
- R₃: wie oben definiert,
- Y =: CH oder N ist,
- R₈ =: ein mit einem Rest R₇ substituierter oder unsubstituierter verzweigter oder unverzweigter Alkylrest mit 1-8 C-Atomen, insbesondere 1-4 C-Atomen, oder ein mit einem Rest R₇ substituierter oder unsubstituierter Aryl- oder Heteroarylrest, oder ein mit einem Rest R₇ substituierter oder unsubstituierter Aralkyl- oder Heteroaralkylrest, oder ein Cyclohexylmethylrest ist und wobei R₇ wie oben beschrieben definiert ist, oder
- P2 =: ein beliebiger α-Azaiminosäurerest folgender Struktur 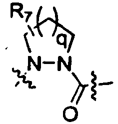
- q =: 0, 1 oder 2, und ein Kohlenstoffatom des Cyclus mit einem Rest R₇ substituiert sein kann, der wie oben definiert ist.

Weitere besonders geeignete Verbindungen sind dadurch gekennzeichnet, dass R₁ ein -CH₂-CO-OH, ein -CH₂-CO-OCH₂CH₃, oder ein Benzylsulfonyl-, ein Methylsulfonyl-, ein Ethylsulfonyl, ein n-Propylsulfonyl oder ein n-Butylsulfonyl-Rest ist. Besonders werden die Benzylsulfonylgruppe und die in den Beispielen verwendeten Gruppen bevorzugt.

Ebenso besonders geeignete Verbindungen sind dadurch gekennzeichnet, dass n = 1 oder 2, insbesondere 2 ist.

Unter dem Begriff Arylrest werden die dem Fachmann geläufigen aromatischen Reste, wie z.B. Phenyl oder Naphtyl. verstanden. Unter dem Begriff Heteroarylrest werden z.B. 5- oder 6-gliedrige Heteroaromaten-Reste verstanden, aber auch kondensierte Heteroaromaten-Reste, wie Chinolin, Purin oder Phenazin. Hinsichtlich der genaueren Beschreibung von Hetereoaromaten und Aromaten wird z.B. auf Römpps Chemie-Lexikon, Thieme 1997 verwiesen.

Besonders geeignete Verbindungen sind dadurch gekennzeichnet, dass der Amino- oder Iminosäurerest mit X und R₂ in der D-Konfiguration vorliegt.

Ebenso sind besonders Verbindungen geeignet, die dadurch gekennzeichnet sind, dass P2 ein Glycin-, ein Serin-, ein Glutaminsäure-, ein Glutaminsäureethylester-, ein Glutaminsäuremethylester oder ein Prolinrest ist.

Ganz besonders bevorzugt bedeutet P2 einen Glycin-, Serin, Glutaminsäure-, Glutaminsäureethylester- oder Glutaminsäureemethylester-Rest.

Insbesondere werden vor allem solche Verbindungen langsamer aus der Zirkulation von Ratten eliminiert, bei denen R₇ eine -COOH oder eine -CH₂-COOH Gruppe ist, oder bei denen P2 ein Glutaminsäurerest ist.

Weitere Beispiele zu bevorzugten Verbindungen bzw. Verbindungsgruppen sind den Ansprüchen zu entnehmen.

Verbindungen der Formel der Struktur (II) oder (III) sind auch Gegenstand der vorliegenden Erfindung.

Pharmazeutisch geeignete bzw. verträgliche Salze sind aufgrund ihrer höheren Wasserlöslichkeit gegenüber den Ausgangs- bzw. Basisverbindungen besonders für medizinische Anwendungen geeignet. Diese Salze müssen ein pharmazeutisch verträgliches Anion oder Kation aufweisen. Geeignete pharmazeutisch verträgliche Säureadditionssalze der erfindungsgemäßen Verbindungen sind z. B. Salze anorganischer Säuren, wie Salzsäure, Bromwasserstoff-, Phosphor-, Methaphosphor-, Salpeter-, Sulfon- und Schwefelsäure oder organischer Säuren, wie z. B. Essigsäure, Benzolfulfon-, Benzoe-, Zitronen-, Ethansulfon-, Fumar-, Glucon-, Glykol-, Isethion-, Milch-, Lactobion-, Malein-, Methansulfon-, Bernstein-, p-Tuluonsulfon-, Wein- und Tri-fluoressigessigsäure. Für medizinische Zwecke wird in besonders bevorzugter Weise das Chlorsalz verwendet. Geeignete phamazeutisch verträgliche basische Salze sind z. B. Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der im folgenden verwendete Begriff "physiologisch funktionelles Derivat" bezeichnet jedes physiologisch verträgliche Derivat einer erfindungsgemäßen Verbindung der Formel I, z. B. einen Ester, der bei Verabreichung an einen Säuger, wie z. B. den Menschen, in der Lage ist, (direkt oder indirekt) eine Verbindung der Formel I oder einen aktiven Metaboliten hiervon zu bilden. Zu den physiologisch funktionellen Derivaten zählen auch Prodrugs der erfindungsgemäßen Verbindung. Solche Prodrugs können in vivo zu einer erfindungsgemäßen Verbindung metabolisiert werden. Diese Prodrugs können selber wirksam sein oder nicht.

Die erfindungsgemäßen Verbindungen können auch in verschiedenen stereoisomeren Formen aber auch in polymorphen Formen vorliegen, z. B. als amorphe und kristalline polymorphe Formen. Alle polymorphen Formen der erfindungsgemäßen Verbindungen sowie die Stereoisomere gehören in den Rahmen der Erfindung und sind ein weiterer Aspekt der Erfindung. Nachfolgend beziehen sich alle Verweise auf Verbindungen gemäß Formel (I) auf Verbindungen) der Formel (I) wie vorstehend beschrieben, sowie Salze, Solvate und physiologisch funktionellen Derivate wie hierin beschrieben.

### Methoden zur Analyse der Verbindungen

### Analytische HPLC

Zur analytischen reversed-phase-HPLC wurde eine HPLC-Anlage LC-10A der Firma Shimadzu, bestehend aus den Teilsystemen CTO-10AS Säulenofen, LC-10AD Pumpen (2 x), DGU-14A Degaser, SIL-10AD Autoinjektor, SCL-10A Systemcontroller, SPD-10A UV-Vis Detektor und einer Säule Luna 5 µm C18(2) 100 Å, 250 x 4,6 mm der Firma Phenomenex, unter Benutzung der zugehörigen Software Shimadzu CLASS-VP, Version 5.3, verwendet. Die Detektion erfolgte bei 220 nm. Als Elutionsmittel dienten Wasser mit 0,1 % TFA (A) und Acetonitril mit 0,1 % TFA (B) bei einer Flussrate von 1 ml/min und einem linearen Gradienten (1% B/min).

### Präparative HPLC

Zur präparativen RP-HPLC wurde eine HPLC-Anlage der Firma Shimadzu, bestehend aus den Teilsystemen LC-8A präparative Pumpen (2 x), DGU-14A Degaser, FRC-10A Fraktionssammler, SCL-10A Systemcontroller, SPD-10A UV-Vis Detektor und einer Säule Luna 5 µm C8(2) 100 Å, 250 x 30,0 mm der Firma Phenomenex, unter Benutzung der zugehörigen Software Shimadzu CLASS-VP, Version 5.3, verwendet. Die Detektion erfolgte bei 220 nm. Als Elutionsmittel dienten ebenfalls Wasser mit 0,1 % TFA (A) und Acetonitril mit 0,1 % TFA (B) bei einer Flussrate von 10 oder 20 ml/min und einem geeigneten Gradienten.

### Massenspektroskopie

Die Massenspektren wurden auf einem ESI-MS LCQ der Firma Finnigan (Bremen, Deutschland), gemessen.

### Dünnschichtchromatographie

Für die Dünnschichtchromatographie wurden Kieselgel-Fertigplatten Adamant UV₂₅₄ der Firma Macherey-Nagel verwendet. Als Laufmittel diente ein Gemisch aus n-Butanol, Eisessig und Wasser (4:1:1). Die Detektion der Verbindungen erfolgte durch UV-Absorption bei 254 nm, außerdem wurden als Sprühreagenzien eine Ninhydrinlösung (300 mg Ninhydrin gelöst in 100 ml n-Butanol und 3 ml Eisessig) und nach Inkubation der DC-Platte in Chloratmosphäre eine o-Tolidinlösung (150 mg o-Tolidin und 2,1 g KI gelöst in 2 ml Eisessig und 148 ml Wasser) verwendet.

### NMR-Spektrometrie

Die NMR-Spekten wurden mit einem Bruker Avance DPX 300 Spektrometer aufgenommen. Dazu wurden die Proben wenn möglich in D₂O, ansonsten in Chloroform-d (CDCl₃) gelöst. Die chemischen Verschiebungen wurden in ppm angegeben und sind auf die Lösungsmittelsignale referiert.

Die vorliegende Erfindung betrifft somit auch die Verwendung der Verbindungen der Formel (I) als Hemmstoffe des Faktors Xa.

Auch eine pharmazeutische Zusammensetzung enthaltend eine Verbindung der Formel (I) ist Gegenstand dieser Erfindung. Die Menge der Verbindung gemäß Formel (I), die erforderlich ist, um den gewünschten biologischen Effekt zu erreichen, ist abhängig von einer Reihe von Faktoren, z. B. der gewählten spezifischen Verbindung, der beabsichtigten Verwendung, der Art der Verabreichung und dem klinischen Zustand des Patienten.

Im allgemeinen liegt die Tagesdosis im Bereich von 0,03 mg bis 100 mg (typischerweise von 3 mg bis 50 mg) pro Tag pro Kilogramm Körpergewicht, z. B. 3-10 mg/kg/Tag. Eine intravenöse Dosis kann z. B. im Bereich von 0,03 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise con 1 ng bis 10 mg pro Milliliter, enthalten. Einzeldosen können z. B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger oder Hilfsstoff in Form einer pharmazeutischen Zusammensetzung vor. Der Träger bzw. Hilfsstoff muss natürlich verträglich sein, in dem Sinn, dass er mit den anderen Bestandteilen der Zusammensetzung kompatibel ist und nicht gesundheitsschädlich für den Patienten ist.

Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind insbesondere solche, die für orale, rektale, topische, perorale (z. B. sublinguale) und parentale (z. B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete Pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver oder Granulate; als Lösung oder Suspension in einer wässrigen oder nicht-wässrigen Flüssigkeit; oder als eine Ölin-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird.

So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose oder Gummi arabicum oder Tragant, und Pastillen, die die Verabreichung einer inerten Basis wie Gelatine und Glycerin oder Saccharose und Gummi arabicum umfassen.

Geeignete pharmazeutische Zusammensetzungen für die parentale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich eine Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Bezüglich der weiteren Formulierung wird auf gängige Handbücher verwiesen.

Die Erfindung betrifft auch Verfahren zur Herstellung von pharmazeutischen Zusammensetzungen, bei denen man eine oder mehrere Verbindungen der allgemeinen Formel (I) mit geeigneten Träger- und Hilfsstoffen (siehe oben) vermischt.

### Verwendete Abkürzungen

- Ac: Acetyl
- Amb: Amidobenzyl
- Ame: Aminomethyl
- aPTT: aktivierte partielle Thromboplastinzeit
- Boc: tert.-Butyloxycarbonyl
- Bz: Benzoyl
- Bzl: Benzyl
- Bzls: Benzylsulfonyl
- DIEA: Diisopropylethylamin
- DCM: Dichlormethan
- DMF: N,N-Dimethylformamid
- HPLC: Hochleistungs-Flüssigkeitschromatographie
- iPr: iso-Propyl
- i.V.: im Vakuum
- LM: Lösungsmittel
- mCPBA: 3-Chlor-Perbenzoesäure
- MS: Massenspektroskopie
- NMM: N-Methylmorpholin
- NMR: Nukleare Magnetresonanzspektroskopie
- PyBOP: Benzotriazol-1-yl-N-oxy-tris(pyrrolidino)phosphoniumhexafluorophosphat
- PT: Prothrombinzeit
- RT: Raumtemperatur
- tBu: tert.-Butyl
- TEA: Triethylamin
- Tfa: Trifluoracetyl
- TFA: Trifluoressigsäure
- THF: Tetrahydrofuran
- TMS-Cl: Trimethylsilylchlorid
- TT: Thrombinzeit

Die Erfindung wird durch die nachfolgenden Beispiele näher erläutert.

### Beispiel 1: Synthese der Inhibitoren

### Inhibitor 1

### Bzls-d-hAla(2-Pyr-NO)-Gly-(2-Aminomethyl-5-Chlor)-Benzylamid x TFA

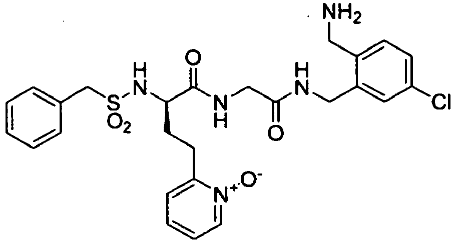

### 1a) 3-(2-Pyridyl)-Propanal

35 ml (0,41 mol) Oxalylchlorid wurden in 700 ml trockenem DCM gelöst und auf -70 °C gekühlt. Zu dieser Lösung wurde ein Gemisch aus 62 ml (0,87 mol) DMSO und 40 ml trockenem DCM über einen Zeitraum von 25 min getropft. Dabei wurde die Temperatur strikt unter -65 °C gehalten (exotherme Reaktion). Nach 15 min Rühren bei -70 °C wurden 50 g (0,36 mol) frisch destillierter 3-(2-Pyridyl)-propanol gelöst in 150 ml trockenem DCM, über einen Zeitraum von maximal 15 min bei unter -65 °C zugetropft. Anschließend wurden 218 ml (0,37 mol) TEA in 40 min zugegeben, danach wurde der Ansatz langsam auf RT erwärmt. Nach Zugabe von 190 ml Wasser lösten sich die gebildeten Salze auf. Die Phasen wurden getrennt, die DCM-Phase wurde im Vakuum eingeengt und anschließend das Produkt destillativ gereinigt (2 mbar, 59-70 °C).
Ausbeute: 33,6 g (0,25 mol, 61 %) farbloses Öl,
DC: R_{f} 0,20.

### 1b) H-d,1-hAla(2-Pyr)-OH

67 g (0,5 mol) 1a wurden mit 18 ml Diethylether versetzt und auf 0 °C gekühlt. 88,4 g (1,65 mol) Ammoniumchlorid wurden in 300 ml Wasser gelöst und langsam zur 2-Pyridyl-3-propanal-Lösung hinzugefügt. Die Mischung wurde mit 74,3 g (1,4 mol) Natriümcyanid, gelöst in 200 ml Wasser, versetzt. Der Ansatz wurde für 4 h bei 0 °C gerührt, anschließend für 4 h auf 50 °C erhitzt und wieder auf RT abgekühlt. Der Ansatz wurde 4 x mit 800 ml Chloroform extrahiert und die vereinigten Chloroformphasen im Vakuum eingeengt. Der Rückstand wurde in 1 l konzentrierter HCl aufgenommen und für 42 h bei RT gerührt und anschließend für 35 h unter Rückfluss gekocht. Das Lösungsmittel wurde im Vakuum entfernt, der Rückstand mehrfach mit Wasser versetzt und der Ansatz im Vakuum wieder eingeengt. Der verbliebene Rückstand wurde in 1,5 l Ethanol gelöst und auf4 °C gekühlt. Die ausgefallenen Salze wurden abgetrennt. Die Mutterlauge wurde eingeengt und in 2 Portionen über einen sauren Ionenaustauscher (Dowex^{®} 50WX8-200, Ammoniumform, 10 cm x 15 cm) gereinigt. Die Elution des Produktes erfolgte mit 0,2 N Ammoniaklösung. Die erhaltenen Fraktionen wurden im Vakuum eingeengt und das Produkt durch Zugabe von Aceton ausgefällt, auf einer Fritte abgesaugt und im Vakuum getrocknet.
Ausbeute: 42 g (0,233 mol, 47 %) hellbrauner Feststoff,
HPLC: 4,9 % B,
DC: R_{f} 0,04.
¹H NMR, 500,13 MHz, D₂O, σ ppm: 8.34 d, breit, ³J{HH} = 5 Hz, 1H; 7,72 ddd ³J{HH} = 7,8 Hz, ³J{HH} = 7,3 Hz, ⁴J{HH} = 1,5 Hz, 1H; 7,27 d ³J{HH} = 7,8 Hz, 1H; 7,23 dd ³J{HH} = 5 Hz, ³J{HH} = 7,3 Hz, 1H; 3,78 t ³J{HH} = 6,3 Hz, 1H; 2,84 m 2H, 2,18 m 2H. ¹³C NMR, 125,75 MHz, D₂O, σ ppm: 174,00; 158,80; 147,84; 138,08; 123,52; 121,99; 54,16; 32,38; 30,23.

### 1c) Bz-d,1-hAla(2-Pyr)-OH

17,7 g (98,22 mmol) H-d,1-hAla(2-Pyr)-OH wurden in 60 ml Dioxan und 60 ml Wasser gelöst und bei 0 °C mit 17,95 ml (103,14 mmol) DIEA versetzt. 11,97 ml (103,14 mmol) Benzoylchlorid wurden in 20 ml Dioxan gelöst und ebenfalls bei 0 °C langsam zugetropft. Der Ansatz wurde über Nacht bei RT gerührt und anschließend das Lösungsmittel im Vakuum entfernt. Der Rückstand wurde in etwas Eisessig angelöst und mit Essigester versetzt. Bei 4 °C kristallisierte das Produkt aus.
Ausbeute: 23,3 g (81,9 mmol, 83 %) weißer kristalliner Feststoff
HPLC: 20,5 % B.

### 1d) Bz-d,1-hAla(2-Pyr)-OMe

23,3 g (81,9 mmol) Bz-d,1-hAla(2-Pyr)-OH wurden in 35 ml trockenem Methanol suspendiert und auf -10 °C gekühlt. Es wurden 8,9 ml (122,85 mmol) Thionylchlorid portionsweise zugegeben und der Ansatz für 30 min bei -10 °C gerührt. Danach wurden weitere 3 ml (40,95 mmol) Thionylchlorid zugegeben. Der Ansatz wurde auf RT erwärmt, über Nacht gerührt und das Lösungsmittel im Vakuum entfernt. Der Rückstand wurde mit 800 ml Essigester gelöst, 2 x mit 200 ml gesättigter NaHCO₃-Lösung gewaschen und mit Na₂SO₄ getrocknet. Das Lösungsmittel wurde im Vakuum entfernt und der Rückstand getrocknet.
Ausbeute: 18,3 g (61,3 mmol, 75 %) amorpher Feststoff
HPLC: 23,7 % B.

### 1e) Bz-d-hAla(2-Pyr)-OMe

6,3 g (21,1 mmol) Bz-d,l-hAla(2-Pyr)-OMe wurden in 200 ml Methanol gelöst und mit 750 ml einer 0,2 N Ammoniumacetatlösung (pH 7,8) versetzt. Der pH wurde mit verdünnter Ammoniaklösung auf 7,5-8 eingestellt. Die Mischung wurde mit 25 mg α-Chymotrypsin (aus Rinderpankreas, Merck, 350 U/mg), angelöst in 1 ml Wasser, versetzt. Der Ansatz wurde für 3 Tage bei 37 °C inkubiert. Dabei wurde der pH-Wert regelmäßig kontrolliert und durch Zugabe von verdünnter Ammoniaklösung konstant auf pH 7,5-8 gehalten. Danach wurde der Ansatz mit Essigsäure auf pH 4 eingestellt, das Lösungsmittel im Vakuum eingeengt und der Rückstand in 2 M Essigsäure gelöst. Durch Zugabe von konzentrierter Ammoniaklösung wurde bei pH 8-9 das Produkt ausgefällt, auf einer Fritte abgesaugt, mit geringen Mengen Ammoniakwasser pH 8,5 gewaschen und im Vakuum getrocknet. Zusätzlich wurde die basische Wasserphase 3 x mit Essigester extrahiert, um noch in der Wasserphase vorhandenes Produkt zu isolieren. Die Essigesterphase wurde mit Na₂SO₄ getrocknet und das Lösungsmittel im Vakuum entfernt.
Ausbeute: 2,65 g (8,9 mmol, 42 %) heller Feststoff
HPLC: 23,7 % B

### 1f) H-d-hAla(2-Pyr)-OH

6 g (2,0 mmol) Bz-d-hAla(2-Pyr)-OMe wurden in 100 ml 6 N HCl gelöst und 20 h unter Rückfluss erhitzt (145 °C Ölbad). Nach Abkühlen auf RT wurde die ausgefallene Benzoesäure abfiltriert, das Lösungsmittel im Vakuum entfernt, der Rückstand noch 2 x in Wasser gelöst und der Ansatz im Vakuum eingeengt. Der Rückstand wurde über einen sauren Ionenaustauscher (Dowex^{®} 50WX8-200, Ammoniumform, 10 cm x 15 cm) gereinigt. Die Elution des Produktes erfolgte mit 0,2 N Ammoniaklösung. Die erhaltenen Fraktionen wurden im Vakuum eingeengt und das Produkt durch Zugabe von Aceton ausgefällt, auf einer Fritte abgesaugt, und im Vakuum getrocknet.
Ausbeute: 1,93 g (1,1 mmol) weißer Feststoff (97 % isomerenrein, Kontrolle mit Marfey's Reagenz)
HPLC: 4,9 % B
DC: R_{f} 0,04.

### 1 g) Bzls-d-hAla(2-Pyr)-OH x TFA

1,97 g (10,9 mmol) H-d-hAla(2-Pyr)-OH wurden in 50 mL DCM vorgelegt, mit 3,04 mL (24,05 mmol) TMS-Cl (Merck) sowie 4,12 mL (24,05 mmol) DIEA (Fluka) versetzt und eine Stunde unter Rückfluß erhitzt. Anschließend wurde der nun völlig klare Ansatz auf Raumtemperatur abgekühlt, mit 2,18 g (11,5 mmol) Benzylsulfonylchlorid (Acros) und 1,96 mL (11,45 mmol) DIEA versetzt. Schließlich wurde der pH Wert mit zusätzlichem DIEA auf 7,5 eingestellt und drei Stunden bei Raumtemperatur gerührt. Das Lösungsmittel wurde im Vakuum entfernt und der Ansatz ohne weitere Vorreinigung mit präparativer reversed-phase HPLC gereinigt und lyophilisiert.
Ausbeute: 1,74 g (3,88 mmol) weißer Feststoff
HPLC: 24,9 % B.
¹H NMR, 300,13 MHz, D₂O, σ ppm: 8,62 d, breit, ³J{HH} = 6,0 Hz, 1H;
8,49, dd ³J{HH} = 7,9 Hz, ³J{HH} = 7,4 Hz, 1H; 7,90 dd ³J{HH} = 7,4 Hz, ³J{HH} = 6,0 Hz, 1H; 7,87 d ³J{HH} = 6,0 Hz, 1H; 7,42 m 5H; 4,49 s 2H; 3,84 dd ³J{HH} = 8,2 Hz, ³J(HH) = 5,2 Hz, 1H; 3,13 t ³J {HH} = 7,6 Hz, 2H, 2,21 m 2H. ¹³C NMR, 75,48 MHz, D₂O, σ ppm: 174,53; 163,08 q ²J{CF} = 35,7 Hz; 155,55; 147,42; 141,19; 131,26; 129,45; 129,33; 128,87; 127,68; 125,55; 116,72 q ¹J{CF} = 292,0 Hz; 59,65; 55,83; 31,56; 29,53.

### 1h) Bzls-d-hAla(2-Pyr)-Gly-OtBu

1,705 g (3,80 mmol) Bzls-d,l-hAla(2-Pyr)-OH x TFA und 0,637g (3,80 mmol) H-Gly-OtBu x HCl wurden in 50 mL DMF gelöst, auf 0° C gekühlt, mit 1,979 g (3,80 mmol) PyBOP und portionsweise mit 1,95 mL (11,4 mmol) DIEA versetzt. Es wurde 1 h bei 0° C und weitere 3 h bei Raumtemperatur gerührt. Anschließend wurde das Lösungsmittel im Vakuum entfernt, der Ansatz in einer minimalen Menge 2 M Essigsäure aufgenommen, mit konzentrierter wässrigen Ammoniaklösung auf pH 8,5 gebracht und drei mal mit Essigester extrahiert. Die kombinierten organischen Phasen wurden über Na₂SO₄ getrocknet, filtriert und im Vakuum eingeengt.
Ausbeute: 3,01 g (Rohprodukt, Öl)
HPLC: 36,9 % B.

### li) Bzls-d-hAla(2-Pyr-NO)-Gly-OtBu

3,01 g (Rohprodukt) 1h wurden in 50 mL DCM gelöst und mit 0,933 g (3,80 mmol) m-CPBA (Fluka, 70 %) oxidiert. Es wurden über einen Zeitraum von 8 h portionsweise weitere 1,51 g (6,12 mmol) m-CPBA nachdosiert. Anschließend wurde das Lösungsmittel im Vakuum entfernt, der Rückstand in einer minimalen Menge 2 M Essigsäure gelöst, mit konzentrierter wässriger Ammoniaklösung auf pH 8,5 gebracht und 3 x mit Essigester extrahiert. Die kombinierten organischen Phasen wurden über Na₂SO₄ getrocknet, filtriert und im Vakuum eingeengt.
Ausbeute: 2,8g Öl (Rohprodukt)
HPLC: 42,8 % B

### 1j) Bzls-d-hAla(2-Pyr-NO)-Gly-OH

2,8 g 1i wurden in 10 mL 90 % TFA gelöst und 45 Minuten geschüttelt. Danach wurde das Lösungsmittel im Vakuum eingeengt und der Ansatz aus Wasser lyophilisiert.
Ausbeute: 1,35 g (3,3 mmol) amorpher Feststoff
HPLC: 26,8 % B

### 1) Bzls-d-hAla(2-Pyr-NO)-Gly-(2-Aminomethyl-5-Chlor)-Benzylamid x TFA

52 mg (0,127 mmol) Bzls-d-hAla(2-Pyr-NO)-Gly-OH und 35 mg (0,127 mmol) H-Amb(2-Boc-Amidomethyl, 5-Cl) (Nelson, T.D. et al., J.Org. Chem. 69 3620 (2004)) wurden in 2 mL DMF gelöst und bei 0 °C mit 66 mg (0,127 mmol) PyBOP sowie 42 µL (0,254 mmol) DIEA versetzt. Der Ansatz wurde 20 Minuten bei 0 °C und weitere 60 Minuten bei Raumtemperatur gerührt. Anschließend wurde das Lösungsmittel im Vakuum entfernt und der verbleibende Rückstand in 1 mL 90 % TFA gelöst. Unter gelegentlich umgeschüttelt wurde der Ansatz für 45 Minuten belassen, anschließend im Vakuum zur Trockne eingeengt und ohne weitere Vorreinigung mit präparativer reversed- phase HPLC gereinigt und lyophilisiert.
Ausbeute: 49 mg (0,072 mmol) lyophilisiertes Pulver
HPLC: 31,3 % B
MS: ber.: 559,17, gef.: 560,2 (M+H)⁺

### Inhibitor 2

### Bzls-d-hAla(2-Pyr-NO)-Ser-(2-Aminomethyl-5-Chlor)-Benzylamid x TFA

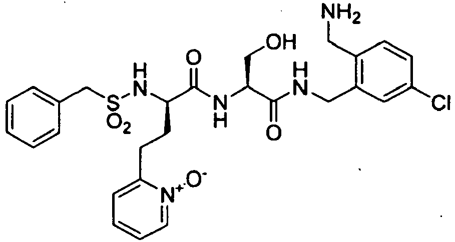

### 2a) Bzls-d,l-hAla(2-Pyr)-Ser(tBu)-OtBu

80,6 mg (0, 80mmol) Bzls-d,l-hAla(2-Pyr)-OH x TFA und 45,6 mg (0,180 mol) H-Ser(tBu)-OtBu x HCl wurden in 4 ml DMF gelöst und bei 0°C mit 93,5 mg (0,180 mmol) PyBop und 123 µl (0,719 mmol) DIEA versetzt. Der Ansatz wurde 20 min bei 0°C und weitere 40 min bei Raumtemperatur gerührt. Anschließend wurde das Lösungsmittel im Vakuum entfernt, der Rückstand mit Essigester aufgenommen, 2 x mit gesättigter NaHCO₃-Lösung gewaschen und über Na₂SO₄ getrocknet. Das Lösungsmittel wurde im Vakuum entfernt.
Ausbeute: 148 mg leicht gelbes Öl (Rohprodukt)
HPLC: 49,47 % B und 49,81 % B (Diastereomere)

### 2b) Bzls-d,l-hAla(2-Pyr-NO)-Ser(tBu)-OtBu

148 mg Bzls-d,l-hAla(2-Pyr)-Ser(tBu)-OtBu (Rohprodukt) wurden in 40ml über Molsieb A4 getrocknetem DCM gelöst, mit 40,3 mg (0,180 mmol) mCPBA (70%) versetzt und bei Raumtemperatur eine Stunde gerührt. Im Laufe einer weiteren Stunde wurden portionsweise weitere 40,3 mg (0,180 mmol) mCPBA zugegeben. Das Lösungsmittel wurde im Vakuum entfernt, der Rückstand mit Essigester aufgenommen, 2 x mit gesättigter NaHCO₃-Lösung und 1 x mit gesättigter NaCl- Lösung gewaschen und über Na₂SO₄ getrocknet. Das Lösungsmittel wurde im Vakuum entfernt.
Ausbeute: 142 mg leicht gelbes Öl (Rohprodukt).
HPLC: 55,39 % B und 55,87 % B (Diastereomere)

### 2c) Bzls-d,l-hAla(2-Pyr-NO)-Ser-OH

142 mg Bzls-d,l-hAla(2-Pyr)-Ser(tBu)-OtBu (Rohprodukt) wurden in 2 ml TFA (90%) gelöst und eine Stunde geschüttelt. Das Lösungsmittel wurde im Vakuum entfernt und das Produkt aus Wasser lyophilisiert.
Ausbeute: 60 mg (0,137 mmol) lyophilisierter Feststoff
HPLC: 25,61 % B und 26,06 % B (Diastereomere)

### 2d) Bzls-d,l-hAla(2-Pyr-NO)-Ser-(2-Boc-Aminomethyl-5-Chlor)-Benzylamid

60 mg Bzls-d,l-hAla(2-Pyr)-Ser(tBu)-OH und 37 mg (0,137 mmol) 2-Boc-Amidomethyl-5-Chlor-Benzylamin wurden in 3 ml DMF gelöst und bei 0°C mit 71 mg PyBop und soviel DIEA versetzt, dass sich ein pH von 8,5 einstellte. Der Ansatz wurde 20 min bei 0°C und weiter 40 min bei Raumtemperatur gerührt. Anschließend wurde das Lösungsmittel im Vakuum entfernt, der Rückstand mit Essigester aufgenommen, 2x mit gesättigter NaHCO₃-Lösung und 1x mit gesättigter NaCl- Lösung gewaschen und über Na₂SO₄ getrocknet. Das Lösungsmittel wurde im Vakuum entfernt.
Ausbeute: 91 mg gelbes Öl (Rohprodukt)
HPLC: 53,175 % B und 53,81 % B (Diastereomere)

### 2) Bzls-d-hAla(2-Pyr-NO)-Ser-(2-Aminomethyl-5-Chlor)-Benzylamid

60 mg 2d (Rohprodukt) wurde in 1 ml TFA (90%) gelöst und 1 h geschüttelt. Das Lösungsmittel wurde im Vakuum entfernt und der Ansatz mit präparativer RP-HPLC gereinigt und lyophilisiert, wobei die Diastereomere getrennt wurden. Die d-Konfiguration der Endverbindung wurde über die Inhibitoraktivität bestimmt.
Ausbeute: 10,5 mg weißer Feststoff
HPLC: 30,33 % B
MS: ber. 589,18; gef.: 590,2 (M+H)⁺

Die Synthese der folgenden Inhibitoren erfolgte nach einer analogen Strategie, die für die Herstellung der Inhibitoren 1 und 2 beschrieben wurde. Dabei wurden bekannte peptidchemische Standardherstellungsverfahren verwendet. Für die Synthese der Inhibitoren 9 und 27 wurde eine modifizierte Synthesestrategie verwendet, die im Detail beschrieben ist. Die letzte Reinigung aller Inhibitoren erfolgte mittels präparativer HPLC.

### Inhibitor 3

### Bzls-l,d-hAla(2-Pyr)-Ser-(2-Aminomethyl-5-Chlor)-Benzylamid x TFA

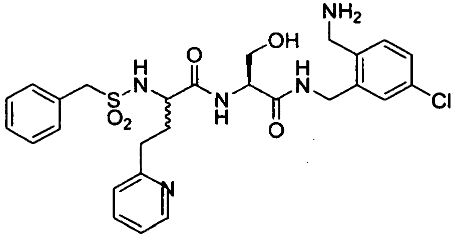

HPLC: 27,2 % B (Diastereomere nicht getrennt)
MS: ber.: 573,2; gef.: 574,2 [M+H]⁺

### Inhibitor 4

### Props-l,d-hAla(2-Pyr-NO)-Gly-(2-Aminomethyl-5-Chlor)-Benzylamid x TFA

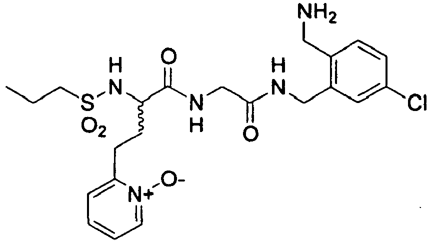

HPLC: 26,9 % B
MS: ber.: 511,2; gef.: 512,2 [M+H]⁺

### Inhibitor 5

### Bzls-l,d-hAla(2-Pyr-NO)-Glu(OMe)-(2-Aminomethyl-5-Chlor)-Benzylamid x TFA

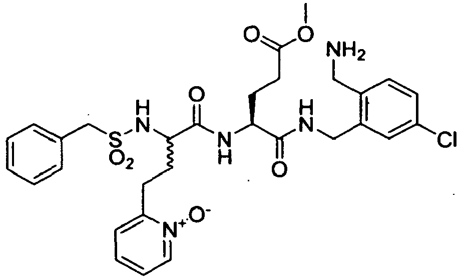

HPLC: Diastereomere bei 34,4 und 34,8 % B
MS: ber.: 645,2 gef.: 646,3 [M+H]⁺

### Inhibitor 6

### Bzls-l,d-hAla(2-Pyr-NO)-Glu-(2-Aminomethyl-5-Chlor)-Benzylamid x TFA

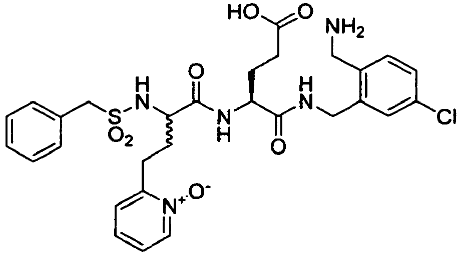

HPLC: 31,8 % B (Diastereomere nicht getrennt)
MS: ber.: 631,2; gef.: 632,3 [M+H]⁺

### Inhibitor 7

### Bzls-d-hAla(2-Pyr-NO)-Pro-(2-Aminomethyl-5-Chlor)-Benzylamid x TFA

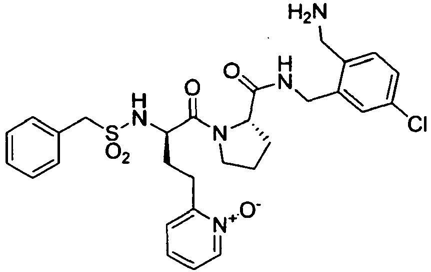

HPLC: 33,5 % B (Diastereomere)
MS: ber.: 599,2; gef.: 600,2 [M+H]⁺

### Inhibitor 8 (Struktur (II))

### 2-Hydroxy-4-Phenyl-butyl-Gly-(2-Aminomethyl-5-Chlor)-Benzylamid x TFA

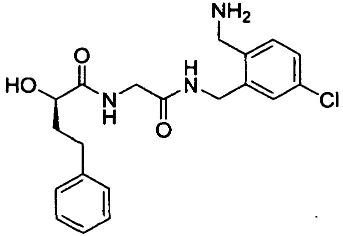

HPLC: 34,4 % B,MS: ber.: 389,1; gef.: 390,1 [M+H]⁺

### Inhibitor 9

### H-l,d-N(CH₂-COOH)hAla(2-Pyr-NO)-Gly-(2-Aminomethyl-5-Chlor)-Benzylamid x 2 TFA

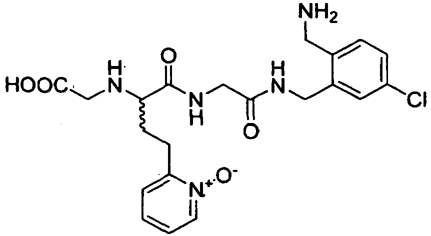

### 9a) Tfa-d,l-hAla(2-Pyr)-OH x TFA

Eine Lösung von 1 Äquivalent H-d,1-hAla(2-Pyr)-OH (0,5 g, 2,77 mmol) in TFA wurde mit einer Eis-Kochsalzmischung auf - 10 °C gekühlt. Anschließend wurden unter Rühren 1,2 Äquivalente Trifluoroessigsäureanhydrid (463 µl, 3,33 mmol) innerhalb weniger Minuten zugetropft. Das Kühlbad wurde entfernt und durch ein 10 °C warmes Wasserbad ersetzt. Nach 30 min wurde überschüssiges Anhydrid und TFA im Vakuum eingeengt und der Rückstand (Öl) mittels präperativer HPLC getrennt.
Ausbeute: 894 mg (82 %)
HPLC 15,5 % B
MS: ber.: 276,07; gef.: 277,04 (M+H)⁺

### 9b) Tfa-d,l-hAla(2-Pyr)-Gly-OtBu

Eine Lösung von 1 Äquivalent 9a (0,5 g, 1,28 mmol) und 1,05 Äquivalente H-Gly-OtBu (225 mg, 1,34 mmol) in 6 ml DMF wurde unter Rühren im Eisbad auf 0° C gekühlt. Zu der gekühlten Lötung wurden 2,7 Äquivalente DIEA (600 µl, 3,47 mmol) und 1,05 Äquivalente PyBOP (0,7 g, 1,34 mmol) zugegeben. Nach 15 min wurde das Eisbad entfernt und unter der Ansatz bei pH 8-9 2 h bei RT gerührt. Anschließend wurde das LM im Vakuum entfernt, der Rückstand in Essigester aufgenommen, jeweils 3 x mit gesättigter NaHCO₃- NaCl-Lösung gewaschen. Die Essigester-Phase wurde über Na₂SO₄ getrocknet und nach Filtration das LM im Vakuum eingeengt. Das verbleibende Öl wurde ohne weitere Aufarbeitung für die nächste Reaktion verwendet.
Ausbeute: 990 mg Rohprodukt (Öl), HPLC: 31,4 % B

### 9c) Tfa-d,l-hAla(2-Pyr-NO)-Gly-OtBu

Das Rohprodukt 9b wurde in DCM gelöst und unter Rühren bei RT mit ca. 1,5 Äquivalenten mCPBA (70 %ig, 475 mg, 1,92 mmol) versetzt. Die HPLC-Kontrolle nach 3 h zeigte noch Ausgangsmaterial, deshalb wurden weitere 0,5 Äquivalente mCPBA nachdosiert und der Ansatz über Nacht gerührt. Das LM wurde im Vakuum eingeengt, der Rückstand in 2 M Essigsäure aufgenommen und der Niederschlag abfiltriert. Anschließend wurde das Filtrat mit wässriger NH₃-Lösung auf pH ∼ 8,5 eingestellt und die Lösung 3 x mit EE extrahiert. Die Essigester-Phase wurde über Na₂SO₄ getrocknet und das LM im Vakuum eingeengt. Das verbleibende Öl wurde ohne weitere Aufarbeitung für die nächste Reaktion verwendet.
Ausbeute: 707 mg Rohprodukt (Öl)
HPLC: 36,0 % B

### 9d) Tfa-d,l-hAla(2-Pyr-NO)-Gly-OH

Das Rohprodukt 9c wurde mit 2 ml 90 % TFA versetzt und 1 h bei RT geschüttelt, mit Wasser verdünnt und lyophilisiert. Das verbleibende Öl wurde ohne weitere Aufarbeitung für die nächste Reaktion verwendet.
Ausbeute: 596 mg Rohprodukt (< 100 %)
HPLC 19,0 % B
MS ber.: 349,09; gef.: 348 ,04 (M-H)⁻

### 9e) Tfa-d,l-hAla(2-Pyr-NO)-Gly-(2-Boc-Amidomethyl-5-Chlor)-Benzylamid

Eine Lösung von ca. 1 mmol Rohprodukt 9d und 1 eq. 2-(Boc-Amidomethyl)-5-Chlor)-Benzylamin (270 mg, 1 mmol) in 3 ml DMF wurde unter Rühren im Eisbad auf 0° C gekühlt. Zu der gekühlten Lösung wurden 1 eq. DIEA (175 µl, 1 mmol) und 1 eq. PyBOP (523 mg, 1 mmol) zugegeben. Nach 15 min wurde das Eisbad entfernt und unter pH-Kontrolle (pH-8-9) 2 h bei RT gerührt. Anschließend wurde das LM im Vakuum entfernt, der Rückstand in Essigester aufgenommen und jeweils 3 x mit gesättigter NaHCO₃- und NaCl-Lösung gewaschen. Die Essigester-Phase wurde über Na₂SO₄ getrocknet, nach Filtration wurde das LM im Vakuum eingeengt. Das verbleibende Rohprodukt wurde mittels präparativer HPLC gereinigt.
Ausbeute: 175 mg
HPLC 47,9 % B

### 9f) H-d,l-hAla(2-Pyr-NO)-Gly-(2-Boc-Amidomethyl-5-Chlor)-Benzylamid x HCl

Eine Lösung von 9e (175 mg, 0,3 mmol) in 1 ml Dioxan und 1 ml 1N NaOH wurde bei 40 °C 3 h gerührt. Anschließend wurde mit 1N HCl neutralisiert, das LM einrotiert und der Rückstand lyophilisiert. Das verbleibende Rohprodukt wurde ohne weitere Aufarbeitung für die nächste Reaktion verwendet.
HPLC 17,7 % B
MS: ber.: 505,21; gef.: 506,1 (M+H)⁺

### 9g) H-d,l-N(CH₂-COOH)-hAla(2-Pyr-NO)-Gly-(2-Boc-Amidomethyl-5-Chlor)-Benzylamid x TFA

Zu einer Lösung des Rohproduktes 9f in 5 ml THF wurden 1,1 Äquivalente K₂CO₃ (45 mg, 0,33 mmol) und 1,1 Äquivalente Bromessigsäureethylester (55 mg, 0,33 mmol) zugegeben und 24 h bei RT gerührt. Der Niederschlag wurde abfiltriert und das LM im Vakuum eingeengt. Anschließend wurde der Rückstand in 2 ml Dioxan aufgenommen und mit 2 ml 1N Na-OH 2 h bei RT gerührt. Anschließend wurde die Mischung mit 1N HCl neutralisiert, das Lösungsmittel im Vakuum eingeengt und der Rückstand mittels präparativer HPLC gereinigt.
Ausbeute: 86 mg (53 %)
HPLC 36,9 % B
MS: ber.: 563,2; gef.: 564,1 (M+H)⁺

### 9h) H-d,l-N(CH₂-COOH)-hAla(2-Pyr-NO)-Gly-(2-Boc-Amidomethyl-5-Chlor)-Benzylamid x 2 TFA

86 mg (0,13 mmol) 9g wurden mit 1 ml 90 % TFA versetzt und 1 h bei RT geschüttelt und anschließend aus H₂O lyophilisiert. Der Rückstand wurde mittels präparativer HPLC gereinigt
Ausbeute: 61 mg (69 %)
C₂₅H₂₆ClF₆N₅O₉: HPLC 17,5 % B
MS: ber.:463,2; gef.: 564,2 (M+H)⁺

### Inhibitor 10

### Bzls-d-hAla(2-Pyr-NO)-Ala-(2-Aminomethyl-5-Chlor)-Benzylamid x TFA

### (Herstellung analog Inhibitor 1 unter Verwendung von H-Ala-OtBu für Schritt h)

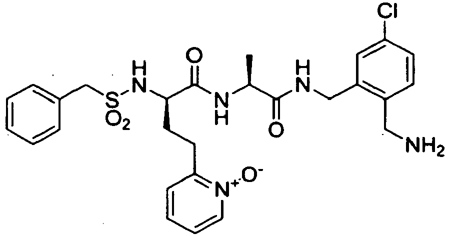

HPLC: 31,8 % B, MS: ber.: 573.18; gef.: 574.2 [M+H]⁺

### Inhibitor 11

### Bzls-d-hAla(2-Pyr-NO)-Glu(OMe)-(2-Aminomethyl-5-Chlor)-Benzylamid x TFA (Herstellung analog Inhibitor 5 und Trennung der Diastereomere im letzten Schritt mittels präparativer HPLC)

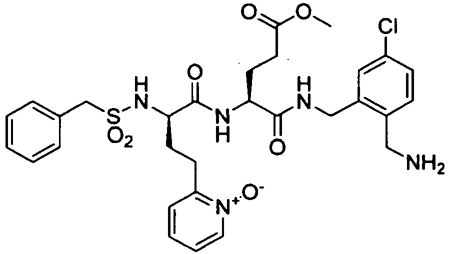

HPLC: 34,4 % B, MS: ber.: 645.2; gef.: 646.3 [M+H]⁺

### Inhibitor 12

### Bzls-d-hAla(2-Pyr-NO)-Dap-(2-Aminomethyl-5-Chlor)-Benzylamid x 2 TFA

### (Herstellung analog Inhibitor 1 unter Verwendung von H-Dap(Boc)-OMe für Schritt h und Verseifung des Methylesters mit LiOH im Schritt j)

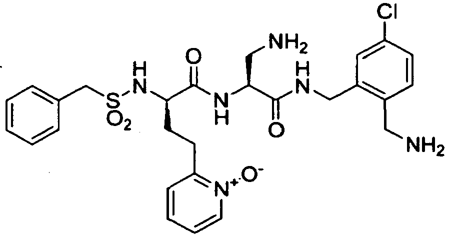

HPLC: 28,4 % B, MS: ber.: 588.19; gef.: 589.2 [M+H]⁺

### Inhibitor 13

### (4-MeOOC-CH₂)-Bzls-d/l-hAla(2-Pyr-NO)-Gly-(2-Aminomethyl-5-Chlor)-Benzylamid x TFA

### (Herstellung analog Inhibitor 1 unter Verwendung von (4-MeOOC)-Bzls-d/l-hAla(2-Pyr)-OH für Schritt h)

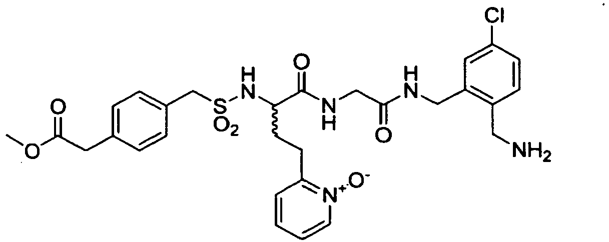

HPLC: 32,2 % B, MS: ber.: 631.19; gef.: 632.3 [M+H]⁺

### Inhibitor 14

### (4-HOOC-CH₂)-Bzls-d/l-hAla(2-Pyr-NO)-Gly-(2-Aminomethyl-5-Chlor)-Benzylamid x TFA

### (Herstellung aus Inhibitor 13 durch Verseifung mit LiOH im letzten Schritt)

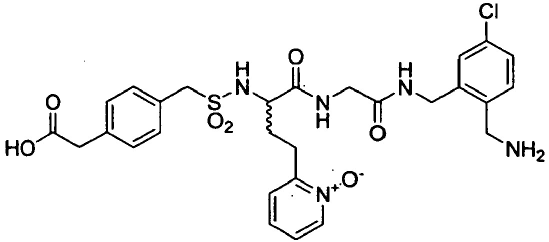

HPLC: 28,2 % B, MS: ber.: 617.17; gef.: 618.3 [M+H]⁺

### Inhibitor 15

### (4-HOOC-CH₂)-Bzls-d/l-hAla(2-Pyr)-Gly-(2-Aminomethyl-5-Chlor)-Benzylamid x TFA

### (Herstellung analog Inhibitor 14 ohne Oxidation des Pyridyl-Stickstoffs)

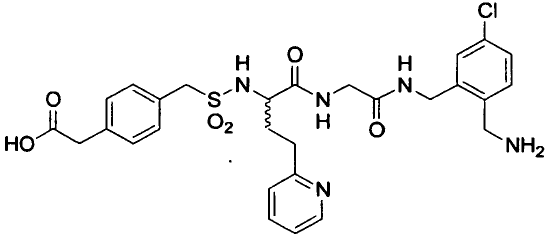

HPLC: 25,6 % B, MS: ber.: 601,18; gef.: 602,3 [M+H]⁺

### Inhibitor 16

### (4-MeOOC-CH₂)- Bzls-d-hAla(2-Pyr-NO)-Ser-(2-Aminomethyl-5-Chlor)-Benzylamid x TFA

### (Herstellung analog Inhibitor 2 unter Verwendung von (4-MeOOC-CH₂)-Bzls-d/l-hAla(2-Pyr)-OH für Schritt a, die Diastereomere wurden im letzten Schritt mittels präparativer HPLC getrennt)

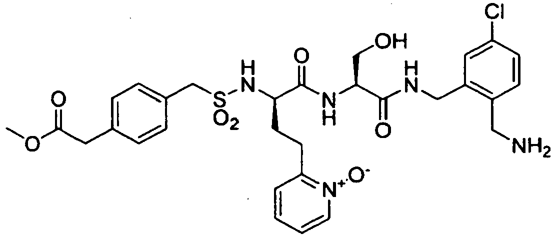

HPLC: 24,9 % B, MS: ber.: 631.19; gef.: 632.2 [M+H]⁺

### Inhibitor 17

### (4-HOOC-CH₂)-Bzls-d-hAla(2-Pyr-NO)-Ser-(2-Aminomethyl-5-Chlor)-Benzylamid x TFA

### (Herstellung aus Inhibitor 16 durch Verseifung mit LiOH im letzten Schritt und Reinigung mittels präparativer HPLC)

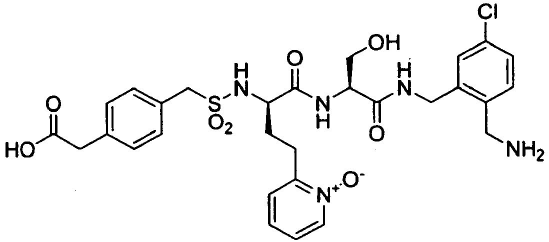

HPLC: 27,4 % B, MS: ber.: 647.18; gef.: 648.3 [M+H]⁺

### Inhibitor 18

### (4-MeOOC-CH₂)-Bzls-d/l-hAla(2-Pyr)-Ser-(2-Aminomethyl-5-Chlor)-Benzylamid x 2 TFA

### (Herstellung analog Inhibitor 16 ohne Oxidation des Pyridyl-Stickstoffs)

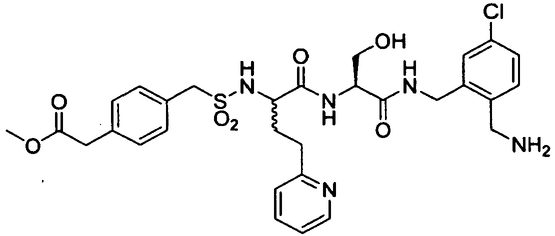

HPLC: 28,5 % B,MS: ber.: 645.2; gef.: 646.3 [M+H]⁺

### Inhibitor 19

### (4-HOOC-CH₂)-Bzls-d-hAla(2-Pyr)-Ser-(2-Aminomethyl-5-Chlor)-Benzylamid x TFA

### (Herstellung aus Inhibitor 18 durch Verseifung mit LiOH im letzten Schritt und Trennung der Diastereomere mittels präparativer HPLC)

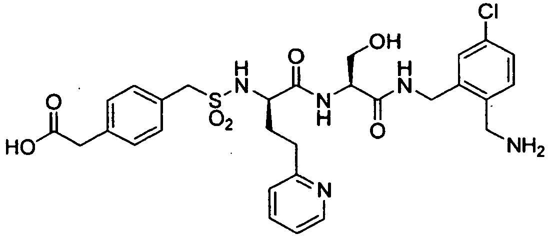

HPLC: 24,9 % B, MS: ber.: 631,19; gef.: 632,3 [M+H]⁺

### Inhibitor 20

### (4-MeOOC)-Bzls-d/l-hAla(2-Pyr-NO)-Gly-(2-Aminomethyl-5-Chlor)-Benzylamid x TFA

### (Herstellung analog Inhibitor 1 unter Verwendung von (4-MeOOC)-Bzls-d/l-hAla(2-Pyr)-OH für Schritt h)

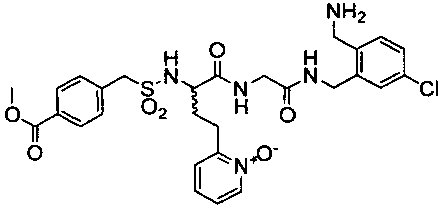

HPLC: 32,16 % B, MS: ber.: 617.17; gef.: 618.2 [M+H]⁺

### Inhibitor 21

### (4-HOOC)-Bzls-d/l-hAla(2-Pyr-NO)-Gly-(2-Aminomethyl-5-Chlor)-Benzylamid x TFA

### (Herstellung aus Inhibitor 20 durch Verseifung mit LiOH im letzten Schritt)

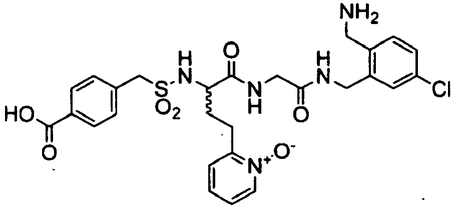

HPLC: 27,13 % B , MS: ber.: 603,16; gef.: 604,2 [M+H]⁺

### Inhibitor 22

### (4-MeOOC)-Bzls-d/l-hAla(2-Pyr)-Gly-(2-Aminomethyl-5 -Chlor)-Benzylamid x 2 TFA

### (Herstellung analog Inhibitor 20 ohne Oxidation des Pyridyl-Stickstoffs)

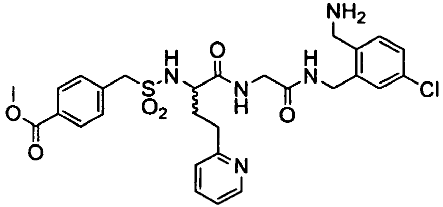

HPLC: 28,96 % B, MS: ber.: 601,18; gef.: 602,2 [M+H]⁺

### Inhibitor 23

### (4-HOOC)-Bzls-d/l-hAla(2-Pyr)-Gly-(2-Aminomethyl-5-Chlor)-Benzylamid x 2 TFA

### (Herstellung aus Inhibitor 22 durch Verseifung mit LiOH)

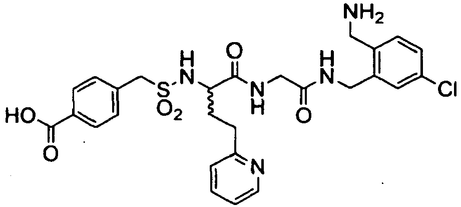

HPLC: 24,58 % B, MS: ber.: 587,16; gef.: 588,2 [M+H]⁺

### Inhibitor 24

### (4-MeOOC)-Bzls-d/l-hAla(2-Pyr-NO)-Ser-(2-Aminomethyl-5-Chlor)-Benzylamid x TFA

### (Herstellung analog Inhibitor 2 unter Verwendung von (4-MeOOC)-Bzls-d/l-hAla(2-Pyr)-OH für Schritt a)

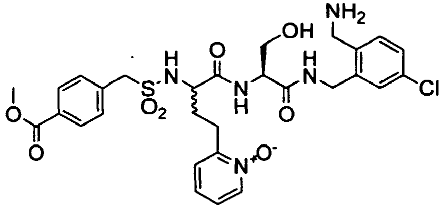

HPLC: 31,36 % B, MS: ber.: 647.18; gef.: 648.2 [M+H]⁺

### Inhibitor 25

### 3-Pyridyl(NO)-CH₂-SO₂-d/l-hAla(2-Pyr-NO)-Gly-(2-Aminomethyl-5-Chlor)-Benzylamid x TFA

### (Herstellung analog Inhibitor 1 unter Verwendung von 3-Pyridyl-CH₂-SO₂-d/l-hAla(2-Pyr)-OH für Schritt h)

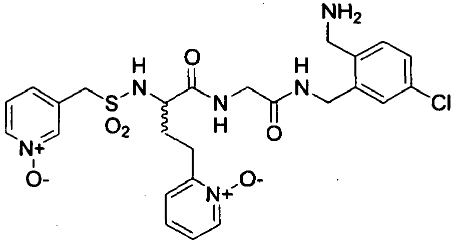

HPLC: 20,7 % B, MS: ber.: 576,16; gef.: 577,2 [M+H]⁺

### Inhibitor 26

### 3-Pyridyl-CH₂-SO₂-d/l-hAla(2-Pyr)-Gly-(2-Aminomethyl-5-Chlor)-Benzylamid x 3 TFA

### (Herstellung analog Inhibitor 25 ohne Oxidation der Pyridylstickstoffe)

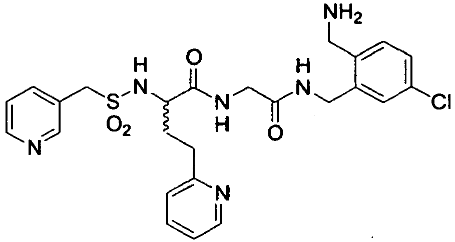

HPLC: 17,8 % B, MS: ber.: 544,17; gef.: 545,2 [M+H]⁺

### Inhibitor 27

### HOOC-CH₂-SO₂-d-hAla(2-Pyr-NO)-Pro-(2-Aminomethyl-5-Chlor)-Benzylamid x TFA

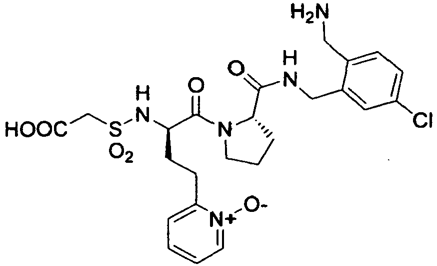

### 27a) Chlorsulfonessigsäure-Isopropylester (Tetrahedron Lett. 2000, 41, 6743)

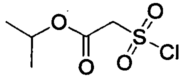

1,166 g (6,3 mmol) kommerziell erhältlicher Chlorsulfonessigsäure-Methylester (Aldrich) wurden in 4 ml trockenem Diethylether gelöst und bei 0 °C mit 485 µl (6,3 mmol) Isopropanol versetzt. Nach 2 h Rühren bei RT wurde das LM im Vakuum eingeengt und der Rückstand ohne weitere Aufarbeitung für den nächsten Schritt verwendet.

### 27b) iPr-OOC-CH₂-SO₂-d/l-hAla(2-Pyr)-OH x HCl

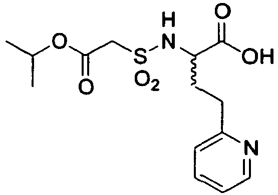

500 mg (2,77 mmol) H-d,1-hAla(2-Pyr)-OH (1b) wurden in 60 mL trockenem DCM suspendiert, mit 830 µL (8,58 mmol) TMS-Cl (Merck) sowie 1,5 mL (8,58 mmol) DIEA (Fluka) versetzt und eine Stunde unter Rückfluß erhitzt. Anschließend wurde der nun völlig klare Ansatz auf 0° C abgekühlt, mit 615 mg (3,05 mmol) 11a und 530 µL (3,05 mmol) DIEA versetzt. Der pH-Wert wurde mit zusätzlichem DIEA auf 7,5-8 eingestellt und weitere 1,5 Stunden bei RT gerührt. Das Lösungsmittel wurde im Vakuum entfernt und der Rückstand in 40 ml Wasser aufgenommen, 3 x mit wenig Essigester gewaschen und die Wasserphase lyophilisiert.
Ausbeute: 2,3 g Rohprodukt mit Salzen
HPLC: 21,2 % B
MS: ber.: 344,1, gef.: 345,1 (M+H)⁺

### 27c) iPr-OOC-CH₂-SO₂-d/l-hAla(2-Pyr)-Pro-OtBu x TFA

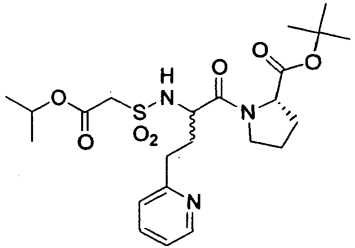

2,04 g (Rohprodukt, ca. 2,42 mmol) 11b und 415 mg (2,42 mmol) H-Pro-OtBu (Bachem) wurden in 15 ml DMF gelöst und bei 0°C mit 1,262 g (2,42 mmol) PyBop und 422 µl (2,42 mmol) DIEA versetzt. Durch Zugabe von DIEA wurde der pH-Wert auf ca. 8-9 eingestellt. Der Ansatz wurde 15 min bei 0°C und weitere 2 h bei RT gerührt. Anschließend wurde das Lösungsmittel im Vakuum entfernt, der Rückstand mit Essigester aufgenommen, 2 x mit gesättigter NaHCO₃-Lösung, 1 x mit gesättigter NaCl-Lösung gewaschen und über Na₂SO₄ getrocknet. Das Lösungsmittel wurde im Vakuum entfernt und der Rückstand (Öl) mittels präperativer RP-HPLC getrennt
Ausbeute: 410 mg Öl
HPLC: 38,0 % B und 39,5 % B (Diastereomere)
MS: ber.: 497,2, gef.: 498,1 (M+H)⁺

### 27d) iPr-OOC-CH₂-SO₂-d/l-hAla(2-Pyr-NO)-Pro-OtBu

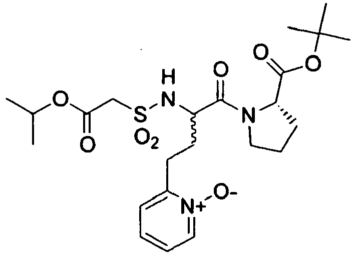

410 mg (0,67 mmol) 11c wurden in 100ml trockenem DCM gelöst, bei 0° C portionsweise mit 500 mg (2,02 mmol) mCPBA (70%) versetzt und anschließend bei RT eine Stunde gerührt. Nach einer Stunde (HPLC Kontrolle) wurden weitere 190 mg (0,767 mmol) mCPBA portionsweise zugegeben. Das Lösungsmittel wurde im Vakuum entfernt, der Rückstand mit Essigester aufgenommen, 2x mit gesättigter NaHCO₃-Lösung, 1x mit gesättigter NaCl- Lösung gewaschen und über Na₂SO₄ getrocknet. Das Lösungsmittel wurde im Vakuum entfernt.
Ausbeute: 800 mg gelbliches Öl (Rohprodukt).
HPLC: 43,3 % B und 45,0 % B (Diastereomere)

### 27e) iPr-OOC-CH₂-SO₂-d-hAla(2-Pyr-NO)-Pro-OH

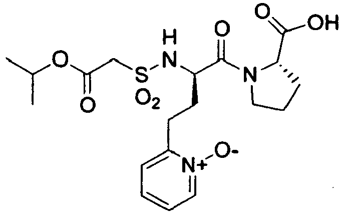

800 mg 11d (Rohprodukt) wurden in 4 ml TFA (90%) gelöst und eine Stunde bei RT geschüttelt. Das Lösungsmittel wurde im Vakuum entfernt und die Diastereomere mittels präparativer HPLC getrennt.
Ausbeute: 70 mg reines Diastereomer (Öl)
HPLC: 29,1 % B

### 27f) iPr-OOC-CH₂-SO₂-d-hAla(2-Pyr-NO)-Pro-(2-Boc-Amidomethyl-5-Chlor)-Benzylamid

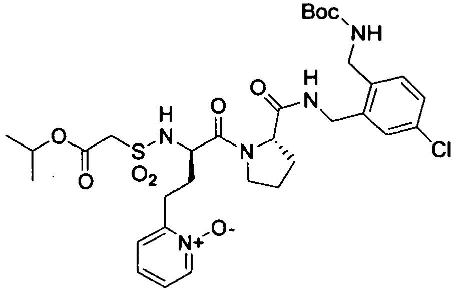

70 mg (0,153 mmol) 11e und 42 mg (0,153 mmol) H-Amb(2-Boc-Amidomethyl,5-Cl) (Nelson, T.D. et al., J.Org. Chem. 69 3620 (2004)) wurden in 5 ml DMF gelöst und bei 0°C mit 79 mg (0,153 mmol) PyBop und 26 µl (0,153 mmol) DIEA versetzt. Der pH-Wert wurde durch weitere Zugabe von DIEA auf 8-9 eingestellt. Der Ansatz wurde 15 min bei 0°C und eine weitere Stunde bei Raumtemperatur gerührt. Anschließend wurde das Lösungsmittel im Vakuum entfernt, der Rückstand mit Essigester aufgenommen, 2 x mit gesättigter NaHCO₃-Lösung, 1 x mit gesättigter NaCl-Lösung gewaschen und über Na₂SO₄ getrocknet.
Ausbeute: 145 mg amorpher Feststoff.
HPLC: 41,59 % B

### 27g) HOOC-Me-SO₂-d-hAla(2-Pyr-NO)-Pro-(2-Aminomethyl-5-Chlor)-Benzylamid x TFA

145 mg 11f (Rohprodukt) wurde in 1 ml TFA (90%) gelöst und 1 h bei Raumtemperatur geschüttelt. Das Lösungsmittel wurde im Vakuum entfernt und der Rückstand mit 3 ml 1 M LiOH und 3 ml MeOH aufgenommen und eine Stunde bei Raumtemperatur geschüttelt. Die Lösung wurde mit 10 % TFA neutralisiert und das Lösungsmittel im Vakuum entfernt. Der Rückstand wurde mittels präparativer RP-HPLC getrennt und das Produkt lyophilisiert.
Ausbeute: 53 mg (weißer Feststoff).
HPLC: 24,48 % B, MS: ber.: 567.16; gef.: 568.2 [M+H]⁺

### Inhibitor 28

### iPr-OOC-CH₂-SO₂d/l-hAla(2-Pyr-NO)-Gly-(2-Aminomethyl-5-Chlor)-Benzylamid x TFA

### (Herstellung analog Inhibitor 27 ohne finale Verseifung des Isopropylesters unter Verwendung von H-Gly-OtBu für Schritt c)

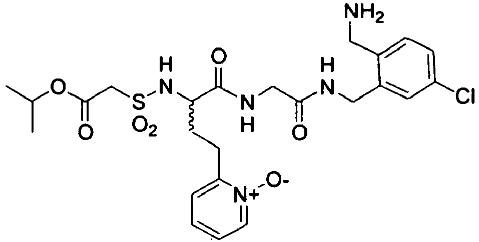

HPLC: 29,7 % B, MS: ber.: 569,17; gef.: 570,2 [M+H]⁺

### Inhibitor 29

### HOOC-CH₂-SO₂-d/l-KAla(2-Pyr-NO)-Gly-(2-Aminomethyl-5-Chlor)-Benzylamid x TFA

### (Herstellung aus Inhibitor 28 durch Verseifung mit LiOH)

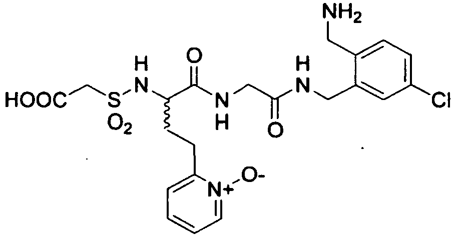

HPLC: 21,4 % B, MS: ber.: 527,12; gef.: 528,3 [M+H]⁺

### Inhibitor 30

### iPr-OOC-CH₂-SO₂-d/l-hAla(2-Pyr)-Gly-(2-Aminomethyl-5-Chlor)-Benzylamid x 2 TFA

### (Herstellung analog Inhibitor 28 ohne Oxidation des Pyridylstickstoffs)

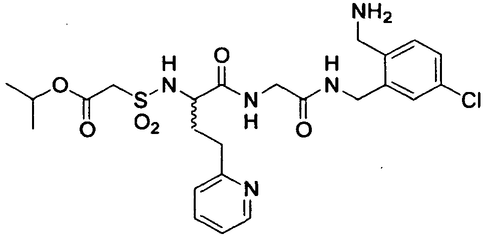

HPLC: 26,7 % B, MS: ber.: 553,18; gef.: 554,3 [M+H]⁺

### Inhibitor 31

### Oxalyl-d/l-hAla(2-Pyr-NO)-Pro-(2-Aminomethyl-5-Chlor)-Benzylamid x TFA

### (Herstellung analog Inhibitor 2 unter Verwendung von Methoxyoxalyl-d/l-hAla (2-Pyr)-OH und H-Pro-OtBu für Schritt a und Verseifung mit LiOH im letzten Schritt)

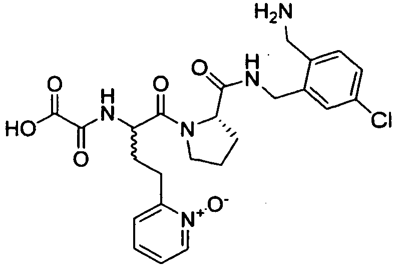

HPLC: 23,9 % B, MS: ber.: 517,17; gef.: 518,2 [M+H]⁺

### Inhibitor 32

### Malonyl-d/l-hAla(2-Pyr-NO)-Gly-(2-Aminomethyl-5-Chlor)-Benzylamid x TFA

### (Herstellung analog Inhibitor 2 unter Verwendung von Ethoxymalonyl-d/l-hAla (2-Pyr)-OH und H-Gly-OtBu für Schritt a und Verseifung mit LiOH im letzten Schritt)

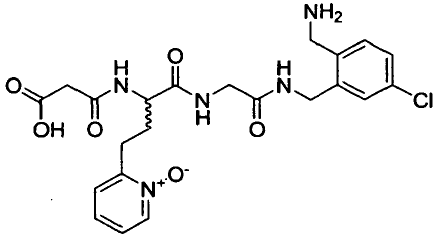

HPLC: 20,4 % B, MS: ber.: 491,16; gef.:492,1 [M+H]⁺

### Inhibitor 33

### H-d-N(CH_{Z}-COOEt)hAla(2-Pyr-NO)-Pro-(2-Aminomethyl-5-Chlor)-Benzylamid x 2 TFA

### (Herstellung analog Inhibitor 9 unter Verwendung von H-Pro-OtBu für Schritt b, die Diastereomere wurden im letzten Schritt mittels präparativer HPLC getrennt)

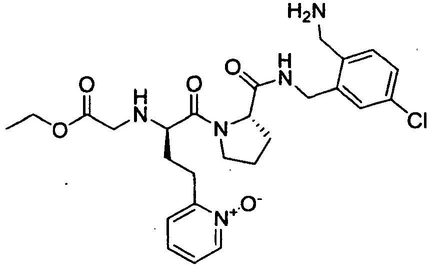

HPLC: 23,23 % B, MS: ber.: 531.22; gef.: 532.3 [M+H]⁺

### Inhibitor 34

### H-d-N(CH₂-COOEt)hAla(2-Pyr)-Pro-(2-Aminomethyl-5-Chlor)-Benzylamid x 3 TFA

### (Herstellung analog Inhibitor 33 ohne Oxidation des Pyridylstickstoffs).

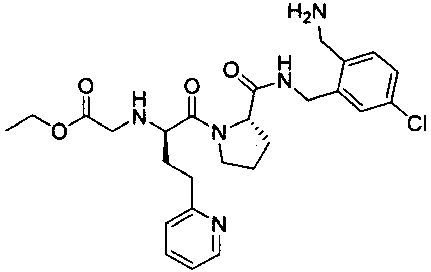

HPLC: 21,7 % B, MS: ber.: 515,23; gef.: 516,2 [M+H]⁺

### Inhibitor 35

### H-d-N(CH₂-COOH)hAla(2-Pyr-NO)-Pro-(2-Aminomethyl-5-Chlor)-Benzylamid x 2 TFA

### (Herstellung aus Inhibitor 33 durch Verseifung mit LiOH im letzten Schritt)

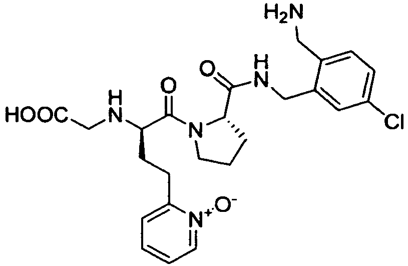

HPLC: 20,36 % B, MS: ber.: 503.19; gef.: 504.3 [M+H]⁺

### Inhibitor 36

### H-d-N(CH₂-COOH)hAla(2-Pyr)-Pro-(2-Aminomethyl-5-Chlor)-Benzylamid x 3 TFA

### (Herstellung analog Inhibitor 35 ohne Oxidation des Pyridylstickstoffs).

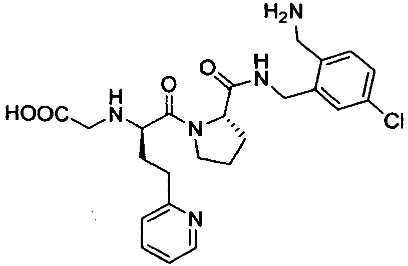

HPLC: 18,0 % B, MS: ber.: 487,19; gef.: 488,2 [M+H]⁺

### Inhibitor 37

### H-d-N(CH₂-COO-Hexyl)hAla(2-Pyr-NO)-Pro-(2-Aminomethyl-5-Chlor)-Benzylamid x 2 TFA

### (Synthese analog Inhibitor 9 unter Verwendung von Bromessigsäurehexylester für Schritt g)

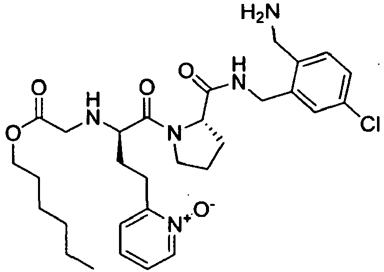

HPLC: 35,55 % B, MS: ber.: 587,29; gef.: 588,4 [M+H]⁺

### Inhibitor 38

### H-d-N(CH₂-COO-Cyclohexyl)hAla(2-Pyr-NO)-Pro-(2-Aminomethyl-5-Chlor)-Benzylamid x 2 TFA

### (Synthese analog Inhibitor 9 unter Verwendung von Bromessigsäurecyclohexylester für Schritt g)

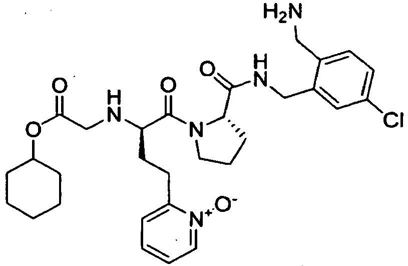

HPLC: 31,47 % B, MS: ber.: 585,27; gef.: 586,3 [M+H]⁺

### Inhibitor 39 (Struktur (III))

### H-d-N(CH₂-COOH)hTyr-Pro-(2-Aminomethyl-5-Chlor)-Benzylamid x 2 TFA

### (Herstellung analog Inhibitor 9 unter Verwendung von Tfa-d-hTyr(tBu)-OH und H-Pro-OtBu für Schritt b)

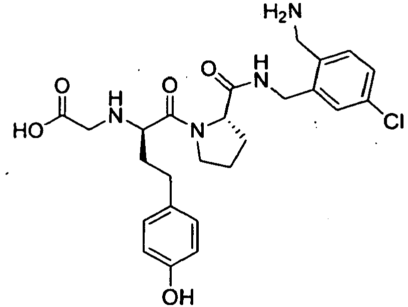

HPLC: 25,2 % B, MS: ber.: 502,2; gef.: 503,2 [M+H]⁺

### Beispiel 2: Enzymkinetische Untersuchungen zur Bestimmung der Hemmwirkung

Die Hemmwirkung der Inhibitoren für Faktor Xa, Thrombin und Plasmin wurden unter Verwendung spezifischer synthetischer chromogener Substrate ermittelt. Die Absorption wurde bei 405 nm mit einem Microplate Reader (iEMS Reader MF 1401, LABSYSTEMS, Helsinki, Finnland) bestimmt und die Berechnung der Kᵢ-Werte erfolgte mit Hilfe eines Computerprogramms durch lineare Regression nach Dixon.
Die Bestimmung für Kᵢ-Werte > 2 nM erfolgte in Tris-Puffer (0,05 M Tris; 0,9 % NaCl; 5 % Ethanol; pH 8,0) auf Mikrotiter-Platten bei 25 °C. Der Inhibitor wurde in Tris-Puffer gelöst, die Substrate (alle Pentapharm Ltd., Basel, CH) in Wasser. Zu 200 µl Inhibitorlösung wurden 25 µl Substratlösung und 50 µl Enzymlösung zugegeben und die Reaktion nach 3-5 min durch Zugabe von 25 µl 50 % Essigsäure abgebrochen (Stürzebecher, J. et al., J. Med. Chem 40, 3091 (1997)). Bei Kᵢ-Werten < 2,0 nM für FXa wurden die Messungen bei reduzierter Enzymkonzentration (100 pM im Ansatz) in Acrylküvetten an einem UV/Vis-Spektralphotometer Specord M 400 (Carl Zeiss, Jena) wiederholt. Es wurden jeweils drei verschiedene Substrat- und fünf verschiedene Inhibitorkonzentrationen gemessen. Die errechneten Kᵢ-Werte entsprechen den Mittelwerten aus mindestens zwei Einzelbestimmungen, deren Einzelwerte nicht mehr als 25 % voneinander abweichen.
Für die Messungen wurden folgende Enzyme und Substrate verwendet:
Humaner Faktor Xa (Enzyme Research Lab., bezogen von Haemochrom Diagnostica GmbH, Essen)
Enzymgehalt: 0,67 µg/ml
Substrat: CH₃OCO-D-Cha-Gly-Arg-pNA (Pefachrome Xa), Konzentration: 4; 2 und 1 mM;
Reaktionszeit: 4 min

### Thrombin (bovin)

Enzymgehalt: 2,5 IE/ml (in 0,9 % NaCl mit 1 % HSA)
Substrat: CH₃SO₂-D-HHT-Gly-Arg-pNA (Pefachrome tPA), Konzentration: 2; 1 und 0,5 mM; Reaktionszeit: 3 min

### Humanes Plasmin (CHROMOGENIX, Milano, Italien)

Enzymgehalt: 500 µg/ml Enzym (in 0,9 % NaCl mit 25 % Glycerol)
Substrat: Tos-Gly-Pro-Lys-pNA (Chromozym PL), Konzentration: 2; 1 und 0,67 mM; Reaktionszeit: 3 min

**Tabelle 1: Hemmung von FXa, Thrombin und Plasmin durch verschiedene Inhibitoren.**

| Inhibitor | Kᵢ (nM) | | |
|---|---|---|---|
| | FXa | Thrombin | Plasmin |
| 1 | 0,095 | 170 | 600000 |
| 2 | 0,059 | 860 | 350000 |
| 3 | 0,25 | 260 | 350000 |
| 4 | 3,4 | 2630 | 280000 |
| 5 | 0,43 | 350 | 130000 |
| 6 | 0,52 | 20500 | 400000 |
| 7 | 0,049 | 0,56 | 8470 |
| 8 | 2200 | 1600 | 540000 |
| 9 | 16 | 125000 | > 1000000 |
| 10 | 0,033 | 170 | 85000 |
| 11 | 0,24 | 180 | 78000 |
| 12 | 0,54 | 750 | 2300 |
| 13 | 0,35 | 1005 | 200000 |
| 14 | 0,36 | 930 | > 1000000 |
| 15 | 0,68 | 140 | > 1000000 |
| 16 | 0,14 | 800 | > 1000000 |
| 17 | 0,18 | 540 | > 1000000 |
| 18 | 0,67 | 430 | > 1000000 |
| 19 | 0,78 | 210 | 100000 |
| 20 | 0,3 | 360 | 140000 |
| 21 | 4,1 | 1300 | 130000 |
| 22 | 1,05 | 100 | 98000 |
| 23 | 11 | 330 | 140000 |
| 24 | 0,48 | 930 | 160000 |
| 25 | 1,4 | 2500 | > 1000000 |
| 26 | 0,34 | 140 | 210000 |
| 27 | 0,08 | 22 | 98000 |
| 28 | 7,6 | 1600 | 305000 |
| 29 | 1,6 | 8300 | > 1000000 |
| 30 | 2,1 | 66 | 290000 |
| 31 | 0,88 | 43 | 80000 |
| 32 | 65 | 140 | n.b. |
| 33 | 0,4 | 4,9 | 240000 |
| 34 | 2,9 | 3,8 | n.b. |
| 35 | 0,4 | 17,2 | 100000 |
| 36 | 1,5 | 3,5 | n.b |
| 37 | 0,92 | n.b. | n.b. |
| 38 | 1,01 | n.b. | n.b. |
| 39 | 8,3 | n.b. | n.b. |

| | | | |
|---|---|---|---|
| n.b. = nicht bestimmt | | | |

### Beispiel 3: Bestimmung der Hemmwirkung der Inhibitoren auf den Prothrombinasekomplex (PTC) und die Gerinnung in humanem Plasma.

### (TT, aPTT, PT)

Die IC₅₀-Werte für die Hemmung des Prothrombinasekomplexes wurden unter Verwendung eines spezifischen chromogenen Substrates ermittelt. Die Bestimmung der Absorption erfolgte mit einem Microplate Reader (siehe oben) bei 405 nm und 37 °C.Die Herstellung des Prothrombinasekomplexes erfolgte durch vorsichtiges Mischen von 250 µl Cephalin (Cephalin-Lyophilisat aus PTT-Reagenz, Roche Diagnostics, Mannheim; gelöst in 5 ml Tris-Puffer A (0,05 M Tris; 0,9 % NaCl; pH 7,5)), 50 µl 0,5 M CaCl₂,25 µl Faktor Xa (human, Haemochrom Diagnostica GmbH, Essen; 0,16 µg/ml),80 µl Faktor Va (human, American Diagnostica, Greenwich, USA; 52 µg/ml) und1845 µl Tris-Puffer B (0,05 M Tris; 0,9 % NaCl; 0,1 % PEG 6000; pH 7,5) auf Eis und anschließende Inkubation für 30 min bei 0 °C. Der Inhibitor wurde in Tris-Puffer B mit5 % Ethanol gelöst. 25 µl Inhibitorlösung wurden mit 45 µl Prothrombinasekomplex für 5 min bei RT inkubiert. Anschließend wurden 30 µl Prothrombin (human, Haemochrom Diagnostica GmbH, Essen; 29 µg/ml) zugegeben, nach 10 min Inkubation bei 37 °C wurde die Reaktion durch Zugabe von 150 µl 0,083 mM EDTA in Puffer B abgestoppt.

Die Aktivität des gebildeten Thrombins wurde nach Zugabe von 50 µl Substrat (H-D-Phe-Pip-Arg-pNA x HCl, S-2238, Haemochrom Diagnostica GmbH, Essen; 0,6 mM) und 200 µl EDTA in Tris-Puffer B zu 25 µl des Inkubationsansatzes chromogen bestimmt.

Der IC₅₀-Wert, d.h. die Inhibitorkonzentration, die eine 50 %ige Hemmung der Thrombinbildung verursacht, wurde graphisch bestimmt. Um zu verhindern, dass durch direkte Thrombinhemmung eine Hemmung des Prothrombinasekomplexes vorgetäuscht wird, wurde auch die direkte Hemmung des generierten Thrombins gemessen (durch Zugabe von 25 µl Tris-Puffer B mit der höchsten Inhibitorkonzentration nach Abstoppen durch EDTA). Bei einer direkten Hemmung des gebildeten Thrombins von > 7 % wurde kein IC₅₀-Wert angegeben. Es wurden jeweils fünf verschiedene Inhibitorkonzentrationen gemessen. Die errechneten IC₅₀-Werte entsprechen den Mittelwerten aus mindestens drei Einzel-bestimmungen, deren Einzelwerte nicht mehr als 25 % voneinander abweichen.

Für die Bestimmung der Gerinnungszeiten wurde humanes Citratplasma verwendet, welches für 10 min bei 3000 U/min zentrifugiert wurde. Die Messungen wurden bei 37 °C mit dem Koagulometer Thrombotrack (Immuno GmbH, Heidelberg) durchgeführt.

Aus der Abhängigkeit der Gerinnungszeit von der Konzentration des Inhibitors wurde der IC₂₀₀-Wert berechnet. Dieser gibt die Inhibitorkonzentration an, die eine Verdopplung der Gerinnungszeit bewirkt. Die errechneten IC₂₀₀-Werte entsprechen den Mittelwerten aus mindestens drei Einzelbestimmungen, deren Einzelwerte nicht mehr als 25 % voneinander abweichen.

### Thrombinzeit (TT)

100 µl humanes Citratplasma wurden mit 50 µl Inhibitorlösung in NaCl (0,9 %; 5 % Ethanol) versetzt und für 2 min bei 37 °C inkubiert. Die Gerinnung wurde durch Zugabe von 50 µl Thrombin (2,5 IE/ml in 0,9 % NaCl mit 1 % HSA) gestartet.

### Prothrombinzeit (PT)

50 µl der Inhibitorlösung in CaCl₂ (0,025 M; 5 % Ethanol) wurden mit 50 µl Thromboplastin (Dade Diagnostika GmbH, Unterschleißheim) für 2 min bei 37 °C inkubiert. Die Gerinnung wurde durch Zugabe von 50 µl humanem Citratplasma gestartet.

### Aktivierte partielle Thromboplastinzeit (aPTT)

50 µl humanes Citratplasma wurden mit 50 µl PTT-Reagenz (Roche Diagnostics, Mannheim) für 3 min bei 37 °C inkubiert. Die Gerinnung wurde durch Zugabe von 50 µl CaCl₂-Lösung (0,025 M; 5 % Ethanol), die den Inhibitor enthielt, gestartet.

**Tabelle 2: Hemmung des Prothrombinasekomplexes (PTC) und gerinnungshemmende Wirksamkeit in humanem Plasma.**

| Inhibitor | PTC IC₅₀(nM) | IC₅₀-Werte (nM) der Gerinnungshemmung | | |
|---|---|---|---|---|
| | | TT | aPTT | PT |
| 1 | 0.48 | 500 | 160 | 140 |
| 2 | 0.51 | 1380 | 270 | 200 |
| 4 | n.b.* | 6500 | 600 | 450 |
| 5 | n.b. | 820 | 220 | 90 |
| 6 | n.b. | >100000 | 750 | 520 |
| 26 | n.b. | 600 | 170 | 350 |
| 27 | n.b. | 110 | 150 | 390 |
| 28 | n.b. | 4200 | 420 | 730 |
| 29 | n.b. | 71000 | 800 | 1300 |
| 31 | n.b. | 140 | 360 | 410 |
| 33 | n.b. | 100 | 180 | 470 |
| 35 | n.b. | 170 | 320 | 610 |

| | | | | |
|---|---|---|---|---|
| *n.b. = nicht bestimmt | | | | |

## Patentansprüche

1. Verbindungen der allgemeinen Formel I: 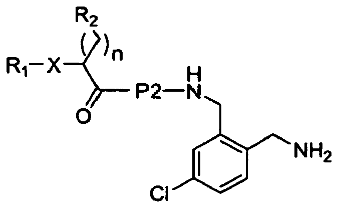 und pharmazeutisch geeignete Salze dieser Verbindungen, bei der
X = NR₃ oder O,
R₃ = H, ein verzweigter bzw. unverzweigter Alkylrest mit 1-6 C-Atomen,
n = 0, 1, 2, 3 oder 4, vorzugsweise 2,
R₁ = H, -CH₂-COOR₄, -SO₂-R₅, -COOR₅ oder CO-R₆;
R₄ = H oder ein verzweigter oder unverzweigter Alkylrest mit 1-6 C-Atomen,
R₅ = ein mit einem Rest R₇ substituierter oder unsubstituierter verzweigter oder unverzweigter Alkylrest mit 1-7 C-Atomen, oder ein mit einem Rest R₇ substituierter oder unsubstituierter Aryl- oder Heteroarylrest, oder ein mit einem Rest R₇ substituierter oder unsubstituierter Aralkyl- oder Heteroaralkylrest, oder ein Cyclohexylmethylrest
R₇ = Halogen, bevorzugt Cl oder F oder CN, ferner NHR₃, NHCO-R₃, -CH₂-NHR₃, NO₂, OR₃, SR₃, -COOR₄ oder -CH₂-COOR₄ und R₃ sowie R₄ wie oben definiert
R₆ = ein mit einem Rest R₇ substituierter oder unsubstituierter verzweigter bzw. unverzweigter Alkylrest mit 1-8 C-Atomen, insbesondere 1-4 C-Atomen, oder Cycloalkyl bzw. ein Cyclohexylmethyl, aber auch -COOR₄, wobei R₄ wie oben definiert ist; und auch R₆ mit R₇ substituiert sein kann, das wie oben definiert ist, und
R₂ = eine beliebige Struktur ausgewählt aus der Gruppe bestehend aus den folgenden Strukturen 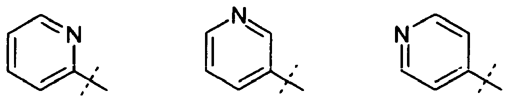 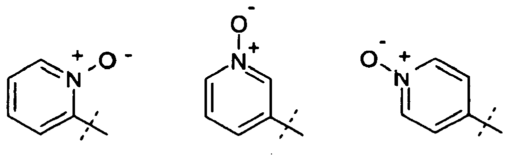
P2 = ein beliebiger natürlicher oder unnatürlicher α-Aminosäurerest folgender Struktur 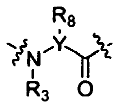 mit
R₃ wie oben definiert,
Y = CH oder N ist,
R₈ = ein mit einem Rest R₇ substituierter oder unsubstituierter verzweigter oder unverzweigter Alkylrest mit 1-8 C-Atomen, insbesondere 1-4 C-Atomen, oder ein mit einem Rest R₇ substituierter oder unsubstituierter Aryl- oder Heteroarylrest, oder ein mit einem Rest R₇ substituierter oder unsubstituierter Aralkyl- oder Heteroaralkylrest, oder ein Cyclohexylmethylrest ist und wobei R₇ wie oben beschrieben definiert ist, oder
P2 = ein beliebiger α-Azaiminosäurerest folgender Struktur 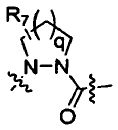
q = 0, 1 oder 2, und ein Kohlenstoffatom des Cyclus mit einem Rest R₇ substituiert sein kann, der wie oben definiert ist.

2. Verbindungen gemäß Anspruch 1, **dadurch gekennzeichnet, dass** R₁ = -CH₂-COOH, -CH₂-COOCH₂CH₃ oder Benzylsulfonyl-, Methylsulfonyl-, Ethylsulfonyl, n-Propylsulfonyl oder n-Butylsulfonyl- ist.

3. Verbindungen gemäß einem der Ansprüche 1 oder 2, **dadurch gekennzeichnet, dass** der Aminosäurerest mit X und R₂ folgende Struktur aufweist:

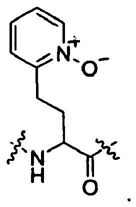

4. Verbindungen gemäß einem der Ansprüche 1 bis 3, **dadurch gekennzeichnet, dass** der Amino- oder Iminosäurerest mit X und R₂ in der D-Konfiguration vorliegt.

5. Verbindungen gemäß einem der Ansprüche 1 bis 3, **dadurch gekennzeichnet, dass** P2 ein Azaglycin-, ein Azaprolin-, ein Serin-, ein Glutaminsäure-, ein Glutaminsäureethylester-, ein Glutaminsäuremethylester- oder ein α,β-Diaminopropionsäurerest ist.

6. Verbindungen gemäß einem der Ansprüche 1 bis 5, **dadurch gekennzeichnet, dass** R₇ eine OH, eine NH₂, eine -COOH, eine -COOCH₂CH₃, eine -CH₂-COOH, oder eine -CH₂-COOCH₂CH₃ Gruppe ist.

7. Verbindungen gemäß einem der Ansprüche 1 bis 6, **dadurch gekennzeichnet, dass** R₁ = -CH₂-COOR₄, vorzugsweise -CH₂-COOC₂H₅. und n = 2 ist.

8. Verbindungen gemäß einem der Ansprüche 1 bis 7, **dadurch gekennzeichnet, dass** R₁ -CH₂COOC₆H₁₁ und/oder P2 ein Alaninrest ist.

9. Verbindungen gemäß einem der Ansprüche 1 bis 8, **dadurch gekennzeichnet, dass**
X = NR₃ oder O,
R₃ = H,
n = 2,
R₁ = H, -CH₂-COOR₄, -SO₂-R₅, -COOR₅ oder CO-R₆;
R₄ = H oder ein verzweigter oder unverzweigter Alkylrest mit 1-6 C-Atomen,
R₅ = ein mit einem Rest R₇ substituierter oder unsubstituierter verzweigter oder unverzweigter Alkylrest mit 1-7 C-Atomen, oder ein mit einem Rest R₇ substituierter oder unsubstituierter Aralkyl- oder Heteroaralkylrest,
R₇ = NHR₃, OR₃ oder -COOR₄ und R₃ sowie R₄ wie oben definiert
R₆ = -COOR₄, wobei R₄ wie oben definiert ist, und
R₂ = gegebenenfalls mit einem Rest R₇ substituierter oder unsubstituierter Aryl- oder Heteroarylrest mit 5-12 Atomen, wobei der Heteroarylrest 1-3 Heteroatome wie N enthalten kann, das, falls R₂ ein Pyridylrest ist, auch als Pyridin-N-Oxid vorliegen kann,
P2 = ein beliebiger natürlicher oder unnatürlicher α-Aminosäurerest folgender Struktur 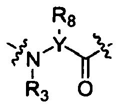 mit
R₃ = wie oben definiert,
Y = CH oder N, und
R₈ = ein mit einem Rest R₇ substituierter oder unsubstituierter verzweigter oder unverzweigter Alkylrest mit 1-8 C-Atomen, insbesondere 1-4 C-Atomen.

10. Verbindungen gemäß einem der Ansprüche 1 bis 9, **dadurch gekennzeichnet, dass**
X = NR₃,
R₃ = H,
n = 2
R₁ = -CH₂-COOR₄, -SO₂-R₅,
R₄ = H oder Ethyl,
R₅ = verzweigter oder unverzweigter Alkylrest mit 1-4 C-Atomen, oder ein mit einem Rest R₇ substituierter oder unsubstituierter Aralkyl- oder Heteroaralkylrest,
R₇ = OR₃ oder -COOR₄ und R₃ sowie R₄ wie oben definiert
R₂ = gegebenenfalls mit einem Rest R₇ substituierter oder unsubstituierter Aryl- oder Heteroarylrest mit 5-12 Atomen, wobei der Heteroarylrest 1-3 Heteroatome wie N enthalten kann, das, falls R₂ ein Pyridylrest ist, auch als Pyridin-N-Oxid vorliegen kann,
P2 = ein beliebiger natürlicher oder unnatürlicher α-Aminosäurerest folgender Struktur 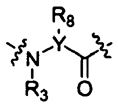 mit
R₃ wie oben definiert,
Y = CH oder N, und
R₈ = ein mit einem Rest R₇ substituierter oder unsubstituierter verzweigter oder unverzweigter Alkylrest mit 1-8 C-Atomen, insbesondere 1-4 C-Atomen.

11. Verbindungen der allgemeinen Formel I: 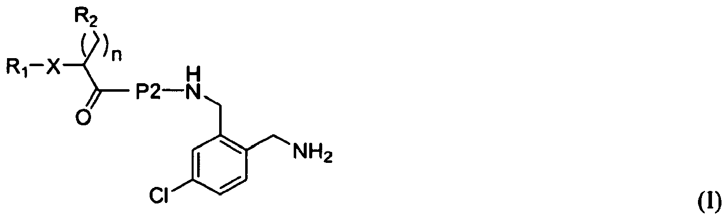 oder pharmazeutisch geeigneter Salze dieser Verbindungen zur Verwendung als Hemmstoffe des Gerinnungsfaktors Xa, bei der
X = NR₃ oder O, R₃ bevorzugt H, aber auch ein verzweigter bzw. unverzweigter Alkylrest mit 1-6 C-Atomen, vor allem Methyl sein kann; mit
n = 0, 1, 2, 3 oder 4, vorzugsweise mit n = 2
R₁ = H, -CH₂-COOR₄, -SO₂-R₅, -COOR₅ oder CO-R₆;
R₄ = H oder ein verzweigter bzw. unverzweigter Alkylrest mit 1-6 C-Atomen, insbesondere Ethyl
R₅ = ein mit einem Rest R₇ substituierter oder unsubstituierter verzweigter oder unverzweigter Alkylrest mit 1-7 C-Atomen, insbesondere 1-4 C-Atomen, oder R₅ ein mit einem Rest R₇ substituierter oder unsubstituierter Aryl- oder Heteroarylrest, oder ein mit einem Rest R₇ substituierter oder unsubstituierter Aralkyl- oder Heteroaralkylrest, oder ein Cyclohexylmethylrest
R₇ = Halogen, bevorzugt Cl oder F oder CN, NHR₃, NHCO-R₃, -CH₂-NHR₃, NO₂, OR₃, SR₃, -COOR₄ oder -CH₂-COOR₄ und R₃ sowie R₄ wie oben definiert
R₆ = ein mit einem Rest R₇ substituierter oder unsubstituierter verzweigter bzw. unverzweigter Alkylrest mit 1-8 C-Atomen, insbesondere 1-4 C-Atomen, oder Cycloalkyl bzw. ein Cyclohexylmethyl, aber auch -COOR₄, wobei R₄ wie oben definiert ist; und auch R₆ mit R₇ substituiert sein kann, das wie oben definiert ist, und
R₂ = eine beliebige Struktur ausgewählt aus der Gruppe bestehend aus den folgenden Strukturen 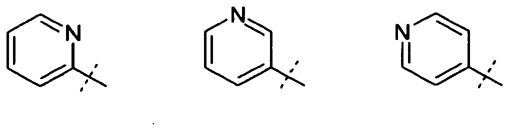 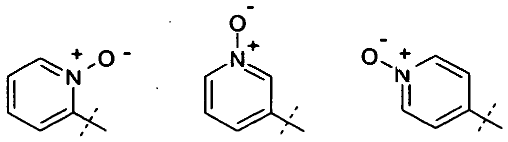
P2 = ein beliebiger natürlicher oder unnatürlicher α-Aminosäurerest bzw. α-Azaaminosäurerest folgender Struktur 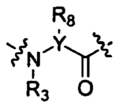 mit
R₃ wie oben definiert, R₈ wie in Anspruch 1 definiert und
Y = CH oder N ist, oder
P2 = ein beliebiger α-Iminosäure- oder α-Azaiminosäurerest folgender Strukturen 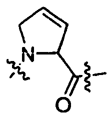 oder vorzugsweise 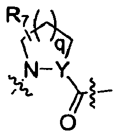 ist,
mit R₇ und Y wie oben definiert und
q = 0, 1 oder 2, und ein Kohlenstoffatom des Cyclus mit einem Rest R₇ substituiert sein kann, der wie oben definiert ist.

12. Verbindungen nach Anspruch 11 zur Verwendung als Hemmstoffe des Gerinnungsfaktors Xa, **dadurch gekennzeichnet, dass** R₁ = -CH₂-COOR₄, vorzugsweise -CH₂-COOC₂H₅, und n = 2 ist.

13. Verbindungen gemäß einem der Ansprüche 11 bis 12 zur Verwendung als Hemmstoffe des Gerinnungsfaktors Xa, **dadurch gekennzeichnet, dass** R₁ -CH₂COOC₆H₁₁ und/oder P2 ein Alaninrest ist.

14. Verbindungen gemäß einem der Ansprüche 11 bis 13 zur Verwendung als Hemmstoffe des Gerinnungsfaktors Xa, **dadurch gekennzeichnet**,
X = NR₃ oder O,
R₃ = H,
n = 2,
R₁ = H, -CH₂-COOR₄, -SO₂-R₅, -COOR₅ oder CO-R₆;
R₄ = H oder ein verzweigter oder unverzweigter Alkylrest mit 1-6 C-Atomen,
R₅ = ein mit einem Rest R₇ substituierter oder unsubstituierter verzweigter oder unverzweigter Alkylrest mit 1-7 C-Atomen, oder ein mit einem Rest R₇ substituierter oder unsubstituierter Aralkyl- oder Heteroaralkylrest,
R₇ = NHR₃, OR₃ oder -COOR₄ und R₃ sowie R₄ wie oben definiert
R₆ = -COOR₄, wobei R₄ wie oben definiert ist, und
R₂ = gegebenenfalls mit einem Rest R₇ substituierter oder unsubstituierter Aryl- oder Heteroarylrest mit 5-12 Atomen, wobei der Heteroarylrest 1-3 Heteroatome wie N enthalten kann, das, falls R₂ ein Pyridylrest ist, auch als Pyridin-N-Oxid vorliegen kann,
P2 = ein beliebiger natürlicher oder unnatürlicher α-Aminosäurerest folgender Struktur 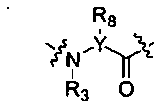 mit
R₃ wie oben definiert, oder ein verzweigter bzw. unverzweigter Alkylrest mit 1-6 C-Atomen, oder ein Prolinrest
Y = CH oder N ist,
R₈ = ein mit einem Rest R₇ substituierter oder unsubstituierter verzweigter oder unverzweigter Alkylrest mit 1-8 C-Atomen, insbesondere 1-4 C-Atomen.

15. Verbindungen gemäß einem der Ansprüche 11 bis 14 zur Verwendung als Hemmstoffe des Gerinnungsfaktors Xa, **dadurch gekennzeichnet, dass**
X = NR₃,
R₃ = H,
n = 2,
R₁ = -CH₂-COOR₄, -SO₂-R₅,
R₄ = H oder Ethyl,
R₅ = verzweigter oder unverzweigter Alkylrest mit 1-4 C-Atomen, oder ein mit einem Rest R₇ substituierter oder unsubstituierter Aralkyl- oder Heteroaralkylrest,
R₇ = OR₃ oder -COOR₄ und R₃ sowie R₄ wie oben definiert
R₂ = gegebenenfalls mit einem Rest R₇ substituierter oder unsubstituierter Aryl- oder Heteroarylrest mit 5-12 Atomen, wobei der Heteroarylrest 1-3 Heteroatome wie N enthalten kann, das, falls R₂ ein Pyridylrest ist, auch als Pyridin-N-Oxid vorliegen kann,
P2 = ein beliebiger natürlicher oder unnatürlicher α-Aminosäurerest außer Glycin und außer am Stickstoffatom alkylierten Glycin folgender Struktur 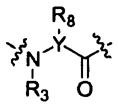 mit
R₃ wie oben definiert,
Y = CH oder N ist, und im Falle von R₈ = H nur N sein darf,
R₈ = ein mit einem Rest R₇ substituierter oder unsubstituierter verzweigter oder unverzweigter Alkylrest mit 1-8 C-Atomen, insbesondere 1-4 C-Atomen ist.

16. Verbindung der Formel der Struktur (II) 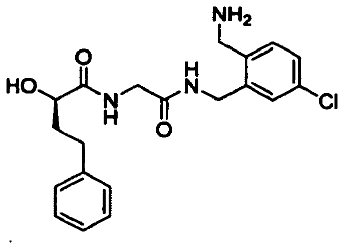 oder 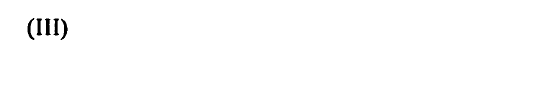 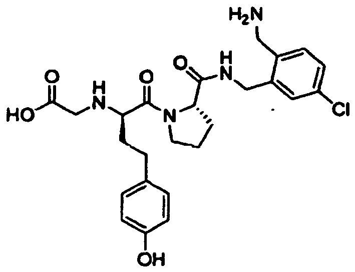 und pharmazeutisch geeignete Salze dieser Verbindung.

## Claims

1. Compounds of the general formula I: 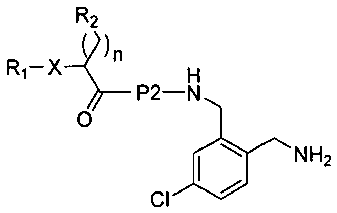 and pharmaceutically suitable salts of these compounds, wherein
X = NR₃ or O,
R₃ = H, a branched or unbranched alkyl radical with 1-6 C atoms,
n = 0, 1, 2, 3 or 4, preferably 2,
R₁ = H, -CH₂-COO₄, -SO₂-R₅, -COOR₅ or CO-R₆;
R₄ = H or a branched or unbranched alkyl radical with 1-6 C atoms,
R₅ = a branched or unbranched alkyl radical with 1-7 C atoms which is unsubstituted or substituted by a radical R₇, or an aryl or heteroaryl radical which is unsubstituted or substituted by a radical R₇, or an aralkyl or heteroaralkyl radical which is unsubstituted or substituted by a radical R₇, or a cyclohexylmethyl radical
R₇ = Halogen, particularly Cl or F or CN, further NHR₃, NHCO-R₃, -CH₂-NHR₃, NO₂, OR₃, SR₃, -COOR₄ or -CH₂-COOR₄ and R₃ as well as R₄ as defined above.
R₆ = a branched or unbranched alkyl radical with 1-8 C atoms, in particular 1-4 C atoms, which is unsubstituted or substituted by a radical R₇, or cycloalkyl or a cyclohexylmethyl, but also -COOR₄, wherein R₄ is as defined above; and R₆ may also be substituted by R₇ which is as defined above, and
R₂ = any structure selected from the group comprising the following structures 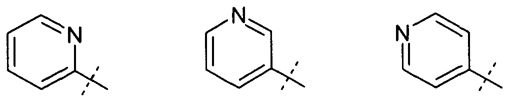 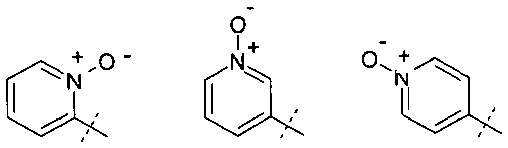
P2 = any natural or unnatural α-amino acid residue of the following structure 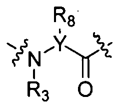 with
R₃ as defined above,
Y = CH or N,
R₈ = a branched or unbranched alkyl radical with 1-8 C atoms, in particular 1-4 C atoms, which is unsubstituted or substituted by a radical R₇, or an aryl or heteroaryl radical which is unsubstituted or substituted by a radical R₇, or an aralkyl or heteroaralkyl radical which is unsubstituted or substituted by a radical R₇, or a cyclohexylmethyl radical, and wherein R₇ is defined as described above, or
P2 = any α-azaimino acid residue of the following structure 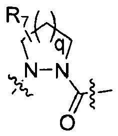
q = 0, 1 or 2, and a carbon atom of the ring may be substituted with a R₇ radical as defined above.

2. The compounds according to claim 1, **characterised in that** R₁ = -CH₂-COOH, -CH₂-COOCH₂CH₃ or benzylsulfonyl-, methylsulfonyl-, ethylsulfonyl, n-propylsulfonyl or n-butylsulfonyl-.

3. The compounds according to claim 1 or 2, **characterised in that** the amino acid residue with X and R₂ has the following structure:

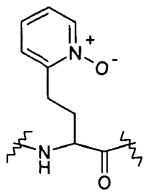

4. The compounds according to any one of claims 1 to 3, **characterized in that** the amino or imino acid residue with X and R₂ is in the D-configuration.

5. The compounds according to any one of claims 1 to 3, **characterized in that** P2 is an azaglycine, an azaproline, a serine, a glutamic acid, a glutamine acid ethylester, glutamine acid methylester or an α, β-diaminopropionic acid residue.

6. The compounds according to any one of claims 1 to 5, **characterized in that** R₇ is a OH, a NH₂ group, a -COOH, a -COOCH₂CH₃, a -CH₂-COOH, or a -CH₂-COOCH₂CH₃ group.

7. The compounds according to any one of claims 1 to 6, **characterized in that** R₁ = - CH₂-COOR₄, preferably -CH₂-COOC₂H₅ and n = 2.

8. The compounds according to any one of claims 1 to 7, **characterized in that** R₁ is - CH₂COOC₆H₁₁ and/or P2 is an alanine residue.

9. The compounds according to any one of claims 1 to 8, **characterized in that**
X = NR₃ or O,
R₃ = H,
n = 2,
R₁ = H, -CH₂-COOR₄, -SO₂-R₅, -COOR₅ or CO-R₆;
R₄ = H or a branched or unbranched alkyl radical with 1-6 C atoms,
R₅ = a branched or unbranched alkyl radical with 1-7 C atoms which is substituted or unsubstituted by a R₇ radical, or a aralkyl or heteroaralkyl radical which is substituted or unsubstituted by a R₇ radical,
R₇ = NHR₃, OR₃ or -COOR₄ and R₃ as well as well as R₄ as defined above
R₆ = -COOR₄, wherein R₄ is as defined above, and
R₂ = aryl or heteroaryl radical with 5-12 atoms which is unsubstituted or optionally substituted by a radical R₇, wherein the heteroaryl radical may comprise 1-3 heteroatoms such as N, which, if R₂ is a pyridyl radical, may also be in the form of pyridine N-oxide,
P2 = any natural or unnatural α-amino acid residue of the following structure 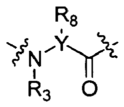 with
R₃ as defined above,
Y = CH or N, and
R₈ = a branched or unbranched alkyl radical with 1-8 C atoms, in particular 1-4 C atoms, which is unsubstituted or substituted by a radical R₇.

10. The compounds according to any one of claims 1 to 9, **characterized in that**
X = NR₃,
R₃ = H,
n = 2
R₁ = -CH₂-COOR₄, -SO₂-R₅,
R₄ = H or ethyl,
R₅ = branched or unbranched alkyl radical with 1-4 C atoms, or an aralkyl or heteroaralkyl radical which is unsubstituted or substituted by a radical R₇,
R₇ = OR₃ or -COOR₄ and R₃ as well as R₄ as defined above.
R₂ = aryl or heteroaryl radical with 5-12 atoms which is unsubstituted or optionally substituted by a radical R₇, wherein the heteroaryl radical may comprise 1-3 heteroatoms such as N, which, if R₂ is a pyridyl radical, may also be in the form of pyridine N-oxide,
P2 = any natural or unnatural α-amino acid residue of the following structure 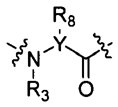 with
R₃ as defined above,
Y = CH or N, and
R₈ = a branched or unbranched alkyl radical with 1-8 C atoms, in particular 1-4 C atoms, which is unsubstituted or substituted by a radical R₇.

11. Compounds of the general formula I: 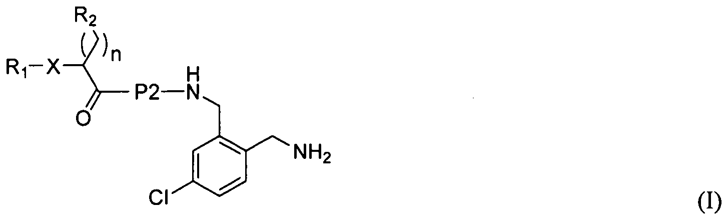 or pharmaceutically suitable salts of these compounds for use as inhibitors of coagulation factor Xa, wherein
X = NR₃ or O, R₃ preferably H, but may also be a branched or unbranched alkyl radical with 1-6 C atoms, especially methyl; with
n = 0, 1, 2, 3 or 4, in particular with n = 2
R₁ = H, -CH₂-COOR₄, -SO₂-R₅, -COOR₅ or CO-R₆;
R₄ = H or a branched or unbranched alkyl radical with 1-6 C atoms, in particular ethyl
R₅ = a branched or unbranched alkyl radical with 1-7 C atoms, in particular 1-4 C atoms, which is unsubstituted or substituted by a radical R₇, or R₅ an aryl or heteroaryl radical which is unsubstituted or substituted by a radical R₇, or an aralkyl or heteroaralkyl radical which is unsubstituted or substituted by a radical R₇, or a cyclohexylmethyl radical
R₇ = Halogen, particularly Cl or F or CN, NHR₃, NHCO-R₃, -CH₂-NHR₃, NO₂, OR₃, SR₃, -COOR₄ or -CH₂-COOR₄ and R₃ as well as R₄ as defined above.
R₆ = a branched or unbranched alkyl radical with 1-8 C atoms, preferably 1-4 C atoms, which is unsubstituted or substituted by a radical R₇, or cycloalkyl or a cyclohexylmethyl, but also -COOR₄, wherein R₄ is as defined above; and R₆ may also be substituted by R₇ which is as defined above, and
R₂ = any structure selected from the group comprising the following structures 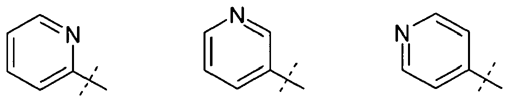 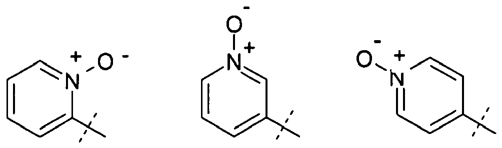
P2 = any natural or unnatural α-amino acid residue or α-azaamino acid residue of the following structure 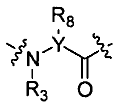 with
R₃ as defined aboved, R₈ as defined in claim 1 and
Y = CH or N, or
P2 = any α-imino acid or α-azaimino acid group of the following structures 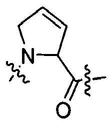 or in particular 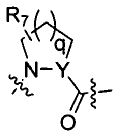
with R₇ and Y as defined above and
q = 0, 1 or 2, and a carbon atom of the ring can be substituted with a R₇ group as defined above.

12. The compounds according to claim 11 for use as inhibitors of coagulation factor Xa, **characterized in that** R₁ = -CH₂-COOR₄, in particular -CH₂-COOC₂H₅, and n = 2.

13. The compounds according to any one of claims 11 to 12 for use as inhibitors of coagulation factor Xa, **characterized in that** R₁ is -CH₂COOC₆H₁₁ and/or P2 is an alanine residue.

14. The compounds according to any one of claims 11 to 13 for use as inhibitors of coagulation factor Xa, **characterized in that**
X = NR₃ or O,
R₃ = H,
n = 2,
R₁ = H, -CH₂-COOR₄, -SO₂-R₅, -COOR₅ or CO-R₆;
R₄ = H or a branched or unbranched alkyl radical with 1-6 C atoms,
R₅ = a branched or unbranched alkyl radical with 1-7 C atoms which is unsubstituted or substituted by a radical R₇, or an aralkyl or heteroaralkyl radical which is unsubstituted or substituted by a radical R₇,
R₇ = NHR₃, OR₃ or -COOR₄ and R₃ as well as R₄ as defined above.
R₆ = -COOR₄, wherein R₄ is as defined above, and
R₂ = aryl or heteroaryl radical with 5-12 atoms which is unsubstituted or optionally substituted by a radical R₇, wherein the heteroaryl radical may comprise 1-3 heteroatoms such as N, which, if R₂ is a pyridyl radical, may also be in the form of pyridine N-oxide,
P2 = any natural or unnatural α-amino acid residue of the following structure 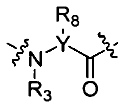 with
R₃ as defined above or a branched or unbranched radical group with 1-6 C atoms, or a proline residue
Y = CH or N,
R₈ = a branched or unbranched alkyl radical with 1-8 C atoms, in particular 1-4 C atoms, which is unsubstituted or substituted by a radical R₇.

15. The compounds according to any one of claims 11 to 14 for use as inhibitors of coagulation factor Xa, **characterized in that**
X = NR₃,
R₃ = H,
n = 2,
R₁ = -CH₂-COOR₄, -SO₂-R₅,
R₄ = H or ethyl,
R₅ = a branched or unbranched alkyl radical with 1-4 C atoms which is unsubstituted or substituted by a radical R₇, or an aralkyl or heteroaralkyl radical which is unsubstituted or substituted by a radical R₇,
R₇ = OR₃ or -COOR₄ und R₃ as well R₄ as defined above
R₂ = aryl or heteroaryl radical with 5-12 atoms which is unsubstituted or optionally substituted by a radical R₇, wherein the heteroaryl radical may comprise 1-3 heteroatoms such as N, which, if R₂ is a pyridyl radical, may also be in the form of pyridine N-oxide,
P2 = any natural or unnatural α-amino acid residue, apart from glycine and apart from glycine alkylated on the nitrogen atom, of the following structure 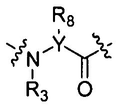 with
R₃ as defined above,
Y = CH or N, and in the case of R₈ = H, may only be N,
R₈ = branched or unbranched alkyl radical with 1-8 C atoms, in particular 1-4 C atoms, which is unsubstituted or substituted by a radical R₇.

16. A compound of the formula of structure (II) 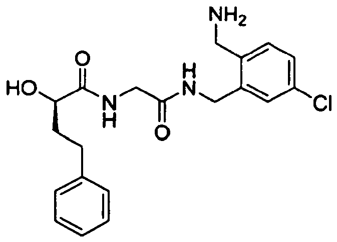 or 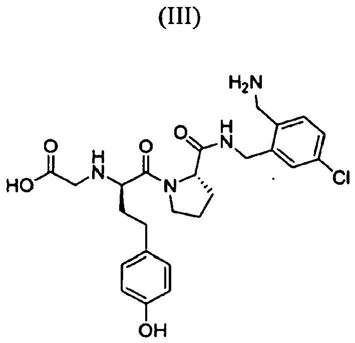 and pharmaceutically suitable salts of this compound.

## Revendications

1. Composés de formule générale I 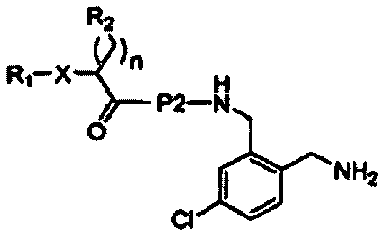 et sels pharmaceutiquement appropriés de ces composés, dans laquelle X = NR₃ ou O,
R₃ = H, un radical alkyle ramifié ou non ramifié comportant 1 à 6 atomes de carbone,
n = 0, 1, 2, 3 ou 4, de préférence 2,
R₁ = H, -CH₂-COOR₄, -SO₂-R₅, -COOR₅ ou CO-R₆ ;
R₄ = H ou un radical alkyle ramifié ou non ramifié comportant 1 à 6 atomes de carbone, R₅ = un radical alkyle comportant 1 à 7 atomes de carbone, ramifié ou non ramifié, substitué ou non substitué par un radical R₇, ou un radical aryle ou hétéroaryle substitué ou non substitué par un radical R₇, ou un radical aralkyle ou hétéroaralkyle substitué ou non substitué par un radical R₇, ou un radical cyclohexylméthyle
R₇ = un atome d'halogène, de préférence Cl ou F ou CN, en outre NHR₃, NHCO-R₃, - CH₂-NHR₃, NO₂, OR₃, SR₃, -COOR₄ ou -CH₂-COOR₄ et R₃ et R₄ sont tels que définis ci-dessus.
R₆ = un radical alkyle comportant 1 à 8 atomes de carbone, ramifié ou non ramifié, substitué ou non substitué par un radical R₇, en particulier 1 à 4 atomes de carbone ou cycloalkyle ou cyclohexylméthyle mais également -COOR₄, R₄ étant tel que défini ci-dessus, et également R₆ peut être substitué par R₇ qui est tel que défini ci-dessus, et
R₂ = une structure quelconque choisie dans le groupe consistant en les structures suivantes 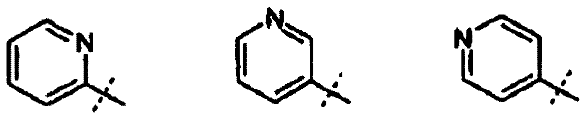 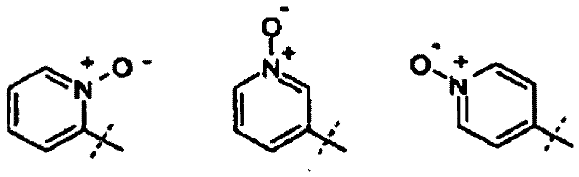 P2 = un radical d'α-amino-acide naturel ou non naturel quelconque de structure suivante 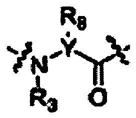 où
R₃ est tel que défini ci-dessus,
Y=CHouN,
R₈ = un radical alkyle comportant 1 à 8 atomes de carbone, ramifié ou non ramifié, substitué ou non substitué par un radical R₇, en particulier 1 à 4 atomes de carbone ou un radical aryle ou hétéroaryle substitué ou non substitué par R₇, ou un radical aralkyle ou hétéroaralkyle substitué ou non substitué par un radical R₇, ou un radical cyclohexylméthyle et R₇ est tel que défini ci-dessus, ou
P2 = un radical d'α-aza-imino-acide quelconque de structure suivante 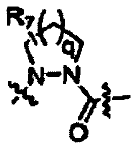 q = 0, 1 ou 2, et un atome de carbone du cycle peut être substitué par un radical R₇ tel que défini précédemment.

2. Composés selon la revendication 1, **caractérisés en ce que** R₁ = -CH₂-COOH, - CH₂-COOCH₂CH₃ ou un groupe benzylsulfonyle, méthylsulfonyle, éthylsulfonyle, n-propylsulfonyle ou n-butylsulfonyle.

3. Composés selon l'une des revendications 1 ou 2, **caractérisés en ce que** le radical d'amino-acide avec X et R₂ est de structure suivante :

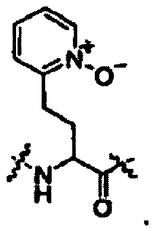

4. Composés selon l'une des revendications 1 à 3, **caractérisés en ce que** le radical d'amino- ou imino-acide avec X et R₂ est de configuration D.

5. Composés selon l'une des revendications 1 à 3, **caractérisés en ce que** P2 est un radical azaglycine, un radical azaproline, un radical sérine, un radical acide glutamique, un radical ester éthylique d'acide glutamique, un radical ester méthylique d'acide glutamique ou un radical acide α, β-diaminopropionique.

6. Composés selon l'une des revendications 1 à 5, **caractérisés en ce que** R₇ est un groupe OH, un groupe NH₂, un groupe -COOH, un groupe -COOCH₂CH₃, un groupe - CH₂-COOH ou un groupe -CH₂-COOCH₂CH₃.

7. Composés selon l'une des revendications 1 à 6, **caractérisés en ce que** R₁ = -CH₂-COOR₄, de préférence -CH₂-COOC₂H₅ et n = 2.

8. Composés selon l'une des revendications 1 à 7, **caractérisés en ce que** R₁ est - CH₂COOC₆H₁₁ et/ou P₂ est un radical alanine.

9. Composés selon l'une des revendications 1 à 8, **caractérisés en ce que**
X = NR₃ ou O,
R₃ = H,
n=2,
R₁ = H, -CH₂-COOR₄, -SO₂-R₅, -COOR₅ ou CO-R₆ ;
R₄ = H ou un radical alkyle ramifié ou non ramifié comportant 1 à 6 atomes de carbone, R₅ = un radical alkyle comportant 1 à 7 atomes de carbone, ramifié ou non ramifié, substitué ou non substitué par un radical R₇, ou un radical aralkyle ou hétéroaralkyle substitué ou non substitué par un radical R₇,
R₇ -NH₃, OR₃ ou -COOR₄ et R₃ et R₄ sont tels que définis ci-dessus R₆ = -COOR₄, R₄ étant tel que défini ci-dessus, et
R₂ = un radical aryle ou hétéroaryle comportant 5 à 12 atomes éventuellement substitué ou non substitué par un radical R₇, le radical hétéroaryle pouvant contenir 1 à 3 hétéroatomes tels que N qui, si R₂ est un radical pyridyle peut se présenter également sous forme de pyridine-N-oxyde,
P2 = un radical d'α-amino-acide naturel ou non naturel quelconque de structure suivante 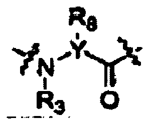 R₃ est tel que défini ci-dessus,
Y = CH ou N, et
R₈ = un radical alkyle ramifié ou non ramifié, substitué ou non substitué par un radical
R₇ et comportant 1 à 8 atomes de carbone, en particulier 1 à 4 atomes de carbone.

10. Composés selon l'une des revendications 1 à 9, **caractérisés en ce que**
X = NR₃,
R₃ = H,
n=2,
R₁ = -CH₂-COOR₄, -SO₂-R₅,
R₄ = H ou un groupe éthyle,
R₅ = un radical alkyle ramifié ou non ramifié comportant 1 à 4 atomes de carbone ou un radical aralkyle ou hétéroaralkyle substitué ou non substitué par R₇,
R₇ = OR₃ ou -COOR₄ et R₃ et R₄ sont tels que définis ci-dessus
R₂ = un radical aryle ou hétéroaryle comportant 5 à 12 atomes de carbone éventuellement substitué par un radical R₇ ou non substitué, le radical hétéroaryle pouvant contenir 1 à 3 hétéroatomes tels que N, qui peut se présenter également, si R₂ est un radical pyridyle, sous forme de pyridin-N-oxyde,
P2 = un radical d'α-amino-acide naturel ou non naturel quelconque de structure suivante 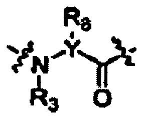 où
R₃ est tel que défini ci-dessus,
Y=CHouN,et
R₈ = un radical alkyle comportant 1 à 8 atomes de carbone, ramifié ou non ramifié, substitué ou non substitué par un radical R₇, en particulier 1 à 4 atomes de carbone.

11. Composés de formule générale 1 : 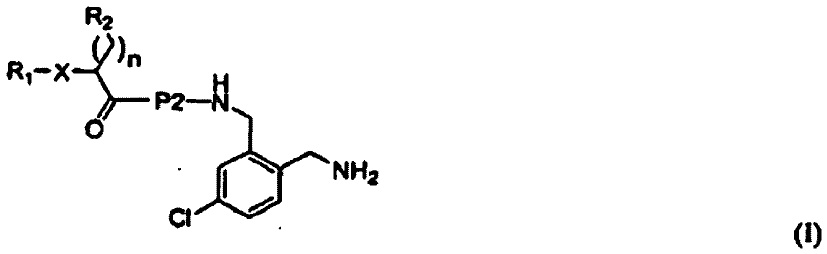 ou des sels de ces composés pharmaceutiquement appropriés, pour l'utilisation comme inhibiteurs du facteur de coagulation Xa, dans laquelle
X = NR₃ ou O, R₃ est de préférence H mais peut être également un radical alkyle ramifié ou non ramifié comportant 1 à 6 atomes de carbone, en particulier un radical méthyle ; où
n = 0, 1, 2, 3 ou 4, de préférence n = 2,
R₁ = H, -CH₂-COOR₄, -SO₂-R₅, -COOR₅ ou CO-R₆ ;
R₄ = H ou un radical alkyle ramifié ou non ramifié comportant 1 à 6 atomes de carbone, en particulier un radical éthyle,
R₅ = un radical alkyle ramifié ou non ramifié, substitué ou non substitué par un radical R₇ et comportant 1 à 7 atomes de carbone, en particulier 1 à 4 atomes de carbone, ou R₅ est un radical aryle ou hétéroaryle substitué ou non substitué par un radical R₇, ou un radical aralkyle ou hétéroaralkyle substitué ou non substitué par un radical R₇, ou un radical cyclohexylméthyle
R₇ = un atome d'halogène, de préférence Cl ou F ou CN, NHR₃, NHCO-R₃, -CH₂-NHR₃, NO₂, OR₃, SR₃, -COOR₄ ou -CH₂-COOR₄ et R₃ et R₄ sont tels que définis ci-dessus R₆ = un radical alkyle ramifié ou non ramifié, substitué ou non substitué par un radical R₇ et comportant 1 à 8 atomes de carbone, en particulier 1 à 4 atomes de carbone, ou cycloalkyle ou cyclohexylméthyle, mais également -COOR₄, dans lequel R₄ est tel que défini ci-dessus ; et R₆ peut également être substitué par R₇ qui est tel que défini ci-dessus, et
R₂ = une structure quelconque choisie dans le groupe consistant en les structures suivantes 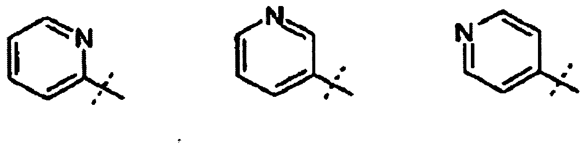 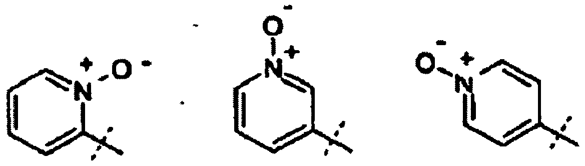 P2 = un radical d'α-amino-acide naturel ou non naturel quelconque ou un radical d'α-aza-imino-acide quelconque de structure suivante 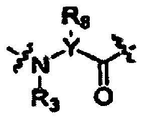 où
R₃ est tel que défini ci-dessus, R₈ est tel que défini dans la revendication 1 et Y=CHouN,ou
P2 = un radical d'α-imino-acide ou un radical d'α-aza-imino-acide quelconque de structure suivante 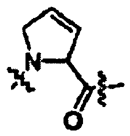 ou de préférence, 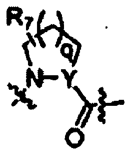 où
R₇ et Y sont tels que définis ci-dessus et
q = 0, 1 ou 2, et un atome de carbone du cycle peut être substitué par un radical R₇ qui est tel que défini précédemment.

12. Composés selon la revendication 11 pour l'utilisation comme inhibiteur du facteur de coagulation Xa, **caractérisés en ce que**
R₁ = -CH₂-COOR₄, de préférence, -CH₂-COOC₂H₅ et n = 2.

13. Composés selon l'une des revendications 11 à 12, pour l'utilisation comme inhibiteurs du facteur de coagulation Xa, **caractérisés en ce que** R₁ est -CH₂-COOC₆H₁₁ et/ou P2 est un radical alanine.

14. Composés selon l'une des revendications 11 à 13, pour l'utilisation comme inhibiteurs du facteur de coagulation Xa, **caractérisés en ce que**
X = NR₃ ou O,
R₃ = H,
n=2
R₁ = H, -CH₂-COOR₄, -SO₂-R₅, -COOR₅ ou CO-R₆ ;
R₄ = H ou un radical alkyle ramifié ou non ramifié comportant 1 à 6 atomes de carbone, R₅ = un radical alkyle comportant 1 à 7 atomes de carbone ramifié ou non ramifié, substitué ou non substitué par un radical R₇, ou un radical aralkyle ou hétéroaralkyle substitué ou non substitué par un radical R₇
R₇ = NHR₃, OR₃ ou -COOR₄ et R₃ et R₄ sont tels que définis ci-dessus,
R₆ = -COOR₄, dans lequel R₄ est tel que défini ci-dessus ; et
R₂ = un radical aryle ou hétéroaryle comportant 5 à 12 atomes éventuellement substitué par un radical R₇ ou non substitué, le radical hétéroaryle pouvant contenir 1 à 3 hétéroatomes tels que N qui, si R₂ est un radical pyridyle, peut se présenter également sous forme de pyridine-N-oxyde,
P2 = un radical d'α-amino-acide naturel ou non naturel quelconque de structure suivante 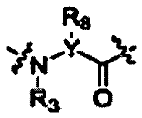 où
R₃ est tel que défini ci-dessus, ou un radical alkyle ramifié ou non ramifié comportant 1 à 6 atomes de carbone,
ou un radical proline
Y = CH ou N,
R₈ = un radical alkyle comportant 1 à 8 atomes de carbone ramifié ou non ramifié, substitué ou non substitué par un radical R₇, en particulier 1 à 4 atomes de carbone.

15. Composés selon l'une des revendications 11 à 14, pour l'utilisation comme inhibiteurs du facteur de coagulation Xa, **caractérisés en ce que**
X = NR₃,
R₃ = H,
n=2,
R₁ = -CH₂-COOR₄, -SO₂-R₅,
R₄ = H ou un groupe éthyle,
R₅ = un radical alkyle ramifié ou non ramifié, comportant 1 à 4 atomes de carbone ou un radical aralkyle ou hétéroaralkyle substitué ou non substitué par un radical R₇,
R₇ = OR₃ ou -COOR₄ et R₃ et R₄ sont tels que définis ci-dessus,
R₂ = un radical aryle ou hétéroaryle comportant 5 à 12 atomes éventuellement substitué par un radical R₇ ou non substitué , le radical hétéroaryle pouvant contenir 1 à 3 hétéroatomes tels que N qui, si R₂ est un radical pyridyle, peut se présenter également sous forme de pyridine-N-oxyde,
P2 est un radical d'α-amino-acide naturel ou non naturel quelconque à l'exception de la glycine et à l'exception de la glycine alkylée sur l'atome d'azote, de structure suivante 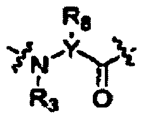 où
R₃ est tel que défini ci-dessus,
Y = CH ou N, et dans le cas où R₈ = H, ne peut être que N,
R₈ = un radical alkyle ramifié ou non ramifié, substitué ou non substitué par un radical R₇ et comportant 1 à 8 atomes de carbone, en particulier 1 à 4 atomes de carbone.

16. Composé de formule structurelle (II) 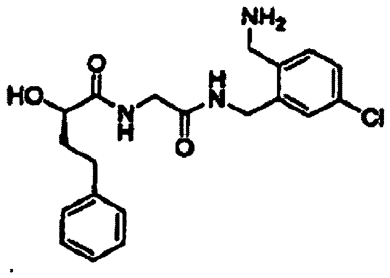 ou 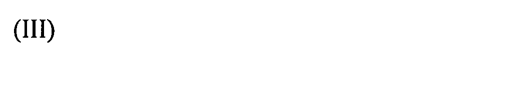 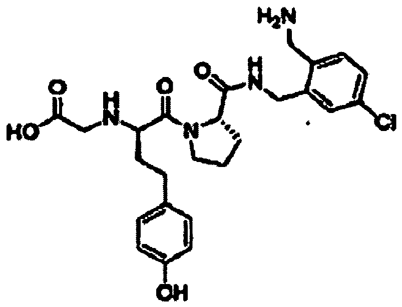 et des sels de ces composés pharmaceutiquement appropriés.
